# EUROPEAN PATENT SPECIFICATION

(11) **EP 3 867 645 B1**
(45) Date of publication and mention of the grant of the patent: **05.06.2024**
(21) Application number: 19873181.2
(22) Date of filing: 25.09.2019
(51) Int. Cl.: G01N 33/564, G01N 33/68, G01B 5/00

(54) **BIOMARKERS FOR A SYSTEMIC LUPUS ERYTHEMATOSUS (SLE) DISEASE ACTIVITY IMMUNE INDEX THAT CHARACTERIZES DISEASE ACTIVITY**
BIOMARKER FÜR KRANKHEITSAKTIVITÄTIMMUNINDEX VON SYSTEMISCHEM LUPUS ERYTHEMATODES (SLE) ZUR CHARAKTERISIERUNG DER KRANKHEITSAKTIVITÄT
BIOMARQUEURS D'INDICE IMMUNITAIRE D'ACTIVITÉ DE MALADIE DU LUPUS ÉRYTHÉMATEUX DISSÉMINÉ (SLE) QUI CARACTÉRISE UNE ACTIVITÉ DE MALADIE

(30) Priority: 18.10.2018 US 201862747455 P
(43) Date of publication of application: 25.08.2021
(73) Proprietor: Oklahoma Medical Research Foundation, Oklahoma City, OK 73104 (US)
(72) Inventor: JAMES, Judith Ann, Edmond, OK 73013 (US); MUNROE, Melissa Elizabeth, Edmond, OK 73012 (US)
(74) Representative: Gill Jennings & Every LLP
(86) International application number: PCT/US2019/052917
(87) International publication number: WO 2020/081204

(56) References cited:
- WO-A1-2017/211896
- WO-A1-2018/140606
- US-A1- 2007 238 094
- MELISSA E. MUNROE ET AL: "Proinflammatory Adaptive Cytokine and Shed Tumor Necrosis Factor Receptor Levels Are Elevated Preceding Systemic Lupus Erythematosus Disease Flare", ARTHRITIS & RHEUMATOLOGY, vol. 66, no. 7, 27 June 2014 (2014-06-27), pages 1888-1899, XP055338974, US ISSN: 2326-5191, DOI: 10.1002/art.38573
- ARRIENS CRISTINA ET AL: "Systemic lupus erythematosus biomarkers: the challenging quest", RHEUMATOLOGY, vol. 56, no. suppl 1, 24 December 2016 (2016-12-24), pages i32-i45, XP055869152, GB ISSN: 1462-0324, DOI: 10.1093/rheumatology/kew407 Retrieved from the Internet: URL:https://www.ncbi.nlm.nih.gov/pmc/artic les/PMC5850341/pdf/kew407.pdf>
- MUNROE, ME et al.: "Innate, Adaptive, and TNF-Superfamily Immune Pathways Inform a Lupus Disease Activity Immune Index That Characterizes Disease Activity in SLE", Arthritis Rheumatology, vol. 70, no. 10, September 2018 (2018-09), pages 1-2, XP055702075,

## Description

### TECHNICAL FIELD OF THE INVENTION

The present invention relates in general to the field of biomarkers for calculating a lupus disease activity immune index that characterizes disease activity in Systemic Lupus Erythematosus (SLE).

### BACKGROUND OF THE INVENTION

Without limiting the scope of the invention, its background is described in connection with Systemic Lupus Erythematosus (SLE).

Systemic autoimmune diseases, including SLE, afflict a significant proportion of the US population. Recent population-based studies reflect a prevalence of 73/100,000 (1, 2), while the Lupus Foundation of America estimates the number of possible SLE patients to be as high as 470/100,000 in routine clinical practice. SLE presents with a constellation of clinical symptoms; disease classification is contingent on meeting 4 of 11 American College of Rheumatology (ACR) criteria (3, 4). More than 90% of affected patients are women age 15-45. Prevalence is higher in minority populations and with lower socioeconomic status (5). Persistently active clinical disease and its treatment place patients at risk for organ damage (6-8), including central nervous system, pulmonary, cardiovascular, and renal damage (9-12), lupus nephritis, and end-stage renal disease (11, 13). Patients with waxing/waning disease and clinically active or quiescent disease are each at risk of clinical disease flare (14, 15).

SLE is a clinically and serologically heterogeneous systemic autoimmune disease that causes significant morbidity and early mortality, especially in young women and minorities. Immune lysregulation in the form of pathogenic autoantibodies and chronic inflammation contributes to a wide range of clinical manifestations, including skin rashes, arthritis, and life-threatening renal and/or central nervous system damage. A number of antinuclear autoantibody (ANA) specificities have been shown to accumulate in SLE patients; use of hydroxychloroquine may abrogate autoantibody accumulation and offset clinical disease activity. Early intervention is an attractive approach to SLE treatment. However, our understanding of pathogenic mechanisms in SLE disease activity is inadequate. Closing this knowledge gap would improve our ability to identify individuals at risk of increased disease activity and permanent organ damage, define windows of opportunity for early intervention, and facilitate the development of pathway-targeted treatments.

Recognition and early treatment to prevent tissue and organ damage is challenging, as signs and symptoms of high disease activity are captured *after* their occurrence. Despite validated clinical disease activity instruments (16-18) and improved treatment strategies, persistently active disease remains a burden for SLE patients (19). Increased morbidity and early mortality associated with treatment required to manage active disease, in particular steroids (20-23), as well as permanent organ damage (24, 25), including renal damage (26), further escalates costs. In addition, long-term use of steroids (23) and other immune-suppressants (27) required to manage disease activity are associated with increased morbidity. The inability to proactively manage clinical disease limits medical care to reactive treatment, precluding proactive strategies of adding or increasing steroid-sparing immune modifying agents (28) to prevent end-organ damage (6-8) and reduce the pathogenic and socioeconomic burdens of SLE (29).

Current biomarkers in SLE have limited utility for forecasting permanent organ damage. Although SLE-associated autoantibody specificities such as anti-dsDNA, anti-spliceosome and anti-Ro/SSA, accumulate in SLE patients, their presence is not sufficient to predict persistent active disease and progression to permanent organ damage. ANAs are also found in sera from patients with other systemic rheumatic diseases, and from healthy individuals who do not go on to develop SLE, including some unaffected family members of SLE patients, and up to 14% of the general population. Because individuals may remain healthy despite being ANA-positive, ANA positivity alone is likely not the sole pathogenic driver of SLE. In addition to ANA positivity, the dysregulation of various immune pathways driven by soluble mediators may contribute to the development of clinical disease. No single factor or mechanism is likely sufficient to explain the complexity and heterogeneity of SLE pathogenesis; thus a multivariate, longitudinal approach is warranted to delineate mechanisms of early disease pathogenesis and discern unique parameters that forecast SLE classification.

Despite clinical trials of a number of directed immune pathway treatments, including the first FDA-approved drug for SLE in over 50 years, Belimumab (30), the vast majority of these studies fail, in part due to lack of understanding the immune pathways dysregulated in a given patient. The need for immune-informed bio-markers as surrogate endpoints for clinical disease activity is becoming more pressing. Administrative burden limits the use of validated SLE clinical disease activity measures in routine practice (31). Validated disease activity instruments, such as the currently used hybrid Systemic Lupus Erythematosus Disease Activity Index (hSLEDAI) (32, 33) and the British Isles Lupus Assessment Group (BILAG) index (17), are labor intensive and require ongoing, specialized training as these clinical instruments are updated (34). Relying solely on physician experience to assess clinical disease activity carries the risk of unwanted variability and negative outcomes (31, 35).

Clinical heterogeneity in SLE underlies the scientific premise that: heterogeneic immune dysregulation underlies clinical disease activity. The inventors have previously shown that patients exhibit immune dysregulation prior to the onset of clinical SLE, amplified in a feed-forward mechanism as patients suffer tissue damage, develop clinical sequelae, and ultimately reach disease classification (36, 37). The inventors also described the accumulation of multiple SLE-associated autoantibodies (AutoAbs) and dysregulated inflammatory and regulatory immune pathways (36, 37). To date, however, the lack of an immune mechanism-informed disease management test in SLE stems from no individual immune pathway-informed biomarker acting as a universal surrogate for either *concurrent* or *future* clinical disease activity (38).

Thus, a need remains for novel methods for detecting, tracking, and evaluating disease activity and progression in patients with classified SLE.

### SUMMARY OF THE INVENTION

The invention is defined in the appended claims.

In one embodiment, the present invention includes a method for characterizing disease activity in a systemic lupus erythematosus (SLE) patient, comprising: (a) obtaining a dataset associated with a blood, serum, plasma or urine sample from the patient, wherein the dataset comprises data representing the level of one or more biomarkers in the blood, serum, plasma or urine sample from each of (b) to (g); (b) assessing the dataset for a presence or an amount of protein expression of at least one innate serum or plasma mediator biomarker selected from: IL-la, IL-1β, IL-1RA, IFN-α, IL-12p70, IL-6, and IL-23p19; (c) assessing the dataset for a presence or an amount of protein expression of at least one adaptive serum or plasma mediator biomarker selected from: IL-2, IFN-γ, IL-5, IL-13, IL-17A, IL-21, IL-10, and TGF-β; (d) assessing the dataset for a presence or an amount of at least one chemokine/adhesion molecule biomarker selected from: IL-8/CXCL8, IP-10/CXCL10, MIG/CXCL9, MIP-1α/CCL3, MIP-1β/CCL4, MCP-1/CCL2, MCP-3/CCL7, and ICAM-1; (e) assessing the dataset for a presence or an amount of at least one soluble TNF superfamily biomarker selected from: TNFRI, TNFRII, TRAIL, TWEAK, CD40L/CD154, BLyS, and APRIL; (f) assessing the dataset for a presence or an amount of the inflammatory mediator biomarker Stem Cell Factor (SCF); (g) assessing the dataset for a presence or an amount at least one SLE-associated autoantibody specificity biomarker selected from: dsDNA, chromatin, RiboP, Ro/SSA, La/SSB, Sm, SmRNP, and RNP; and (h) calculating a Lupus Disease Activity Immune Index (LDAII) score. In one aspect, at least 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, or 39 biomarkers are used in the calculation of the LDAII. The dataset is: log transformed; standardized; weighted by Spearman r correlation to the autoantibody specificities in the second dataset, and a summation of soluble protein markers equals an LDAII score. In another aspect, the performance of the at least one immunoassay comprises: obtaining the first sample, wherein the first sample comprises the protein markers; contacting the first sample with a plurality of distinct reagents; generating a plurality of distinct complexes between the reagents and markers; and detecting the complexes to generate the data. In another aspect, the at least one immunoassay comprises a multiplex assay. In another aspect, the LDAII divides a level of severity or progression of the SLE into clinically active (CA) or quiescent (CQ) disease that is either serologically (dsDNA binding and low complement) active (SA) or serologically quiescent (SQ). In another aspect, the LDAII score distinguishes between active and low lupus disease activity. A treatment may be administered to the patient prior to reaching clinical disease classification after determining that the patient has the prognosis for transitioning to classified SLE, wherein the treatment comprises at least one of: hydroxychloroquine (HCQ), belimumab, a nonsteroidal anti-inflammatory drug, a steroid, or a disease-modifying anti-rheumatic drug (DMARD).

In another embodiment, the present invention includes a method of evaluating disease activity and progression of Systemic Lupus Erythematosus (SLE) clinical disease in a patient comprising: obtaining a blood, serum, plasma or urine sample from the patient; performing at least one immunoassay on a sample from the patient to generate a dataset comprising at least one biomarker from each of (1) to (6): (1) assessing the dataset for a presence or an amount of protein expression of at least one innate serum or plasma mediator biomarker selected from: IL-la, IL-1β, IL-1RA, IFN-α, IL-12p70, IL-6, and IL-23p19; (2) assessing the dataset for a presence or an amount of protein expression of at least one adaptive serum or plasma mediator biomarker selected from: IL-2, IFN-γ, IL-5, IL-13, IL-17A, IL-21, IL-10, and TGF-β; (3) assessing the dataset for a presence or an amount of at least one chemokine/adhesion molecule biomarker selected from: IL-8/CXCL8, IP-10/CXCL10, MIG/CXCL9, MIP-1α/CCL3, MIP-1β/CCL4, MCP-1/CCL2, MCP-3/CCL7, and ICAM-1; (4) assessing the dataset for a presence or an amount of at least one soluble TNF superfamily biomarker selected from: TNFRI, TNFRII, TRAIL, TWEAK, CD40L/CD154, BLyS, and APRIL; (5) assessing the dataset for a presence or an amount of the inflammatory mediator biomarker SCF; and (6) assessing the dataset for a presence or an amount at least one SLE-associated autoantibody specificity biomarker selected from: dsDNA, chromatin, RiboP, Ro/SSA, La/SSB, Sm, SmRNP, and RNP; and calculating an LDAII score. In one aspect, at least 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, or 39 biomarkers are used in the calculation of the LDAII. The dataset is: log transformed; standardized; weighted by Spearman r correlation to the autoantibody specificities in the second dataset, and a summation of the soluble protein markers equals a Lupus Disease Activity Immune Index (LDAII) score. In another aspect, performance of the at least one immunoassay comprises: obtaining the first sample, wherein the first sample comprises the protein markers; contacting the first sample with a plurality of distinct reagents; generating a plurality of distinct complexes between the reagents and markers; and detecting the complexes to generate the data. In another aspect, the at least one immunoassay comprises a multiplex assay. In another aspect, the LDAII divides a level of severity or progression of the SLE into clinically active (CA) or quiescent (CQ) disease that is either serologically (dsDNA binding and low complement) active (SA) or serologically quiescent (SQ). In another aspect, the LDAII score distinguishes between active and low lupus disease activity. A treatment may be administered to the SLE patient prior to reaching clinical disease classification after determining that the patient has the prognosis for transitioning to classified SLE, wherein the treatment comprises at least one of: hydroxychloroquine (HCQ), belimumab, a nonsteroidal anti-inflammatory drug, a steroid, or a disease-modifying anti-rheumatic drug (DMARD). In another aspect, obtaining the first dataset associated with the sample comprises obtaining the sample and processing the sample to experimentally determine the first dataset, or wherein obtaining the first dataset associated with the sample comprises receiving the first dataset from a third party that has processed the sample to experimentally determine the first dataset. In another aspect, an increase in the SCF, TNFRII, and MCP-1 biomarkers are indicative of renal organ involvement.

In another embodiment, the present invention includes a method of calculating a Lupus Disease Activity Immune Index (LDAII) by measuring expression levels of a set of biomarkers in a subject comprising: determining biomarker measures of a set of biomarkers by immunoassay in a physiological sample, wherein the biomarkers are peptides, proteins, peptides bearing post-translational modifications, proteins bearing post-translational modification, or a combination thereof; wherein the physiological sample is whole blood, blood plasma, blood serum, or a combination thereof; wherein the set of biomarkers comprise a dataset of measurements selected from at least one from each of following categories of biomarkers: a presence or an amount of protein expression of at least one innate serum or plasma mediator biomarker dataset selected from: IL-1α, IL-1β, IL-1RA, IFN-α, IL-12p70, IL-6, and IL-23p19; a presence or an amount of protein expression of at least one adaptive serum or plasma mediator biomarker dataset selected from: IL-2, IFN-γ, IL-5, IL-13, IL-17A, IL-21, IL-10, and TGF-β; a presence or an amount of at least one chemokine/adhesion molecule biomarker dataset selected from: IL-8/CXCL8, IP-10/CXCL10, MIG/CXCL9, MIP-1α/CCL3, MIP-1β/CCL4, MCP-1/CCL2, MCP-3/CCL7, and ICAM-1; a presence or an amount of at least one soluble TNF superfamily biomarker dataset selected from: TNFRI, TNFRII, TRAIL, TWEAK, CD40L/CD154, BLyS, and APRIL; a presence or an amount of the inflammatory mediator biomarker SCF; and a presence or an amount at least one SLE-associated autoantibody specificity biomarker dataset selected from: dsDNA, chromatin, RiboP, Ro/SSA, La/SSB, Sm, SmRNP, and RNP; and calculating an LDAII score, whereby the dataset is: log transformed; standardized; weighted by Spearman r correlation to the autoantibody specificities in the second dataset, and a summation of the soluble protein markers equals an LDAII score. In another aspect, the method further comprises classifying the sample with respect to the presence or development of Systemic Lupus Erythematosus (SLE) into clinically active (CA) or quiescent (CQ) disease that is either serologically (dsDNA binding and low complement) active (SA) or serologically quiescent (SQ) in the subject using the set of biomarker measures in a classification system, wherein the classification system is a machine learning system comprising classification and regression trees selected from the group consisting of Fisher's exact test, Mann-Whitney test, Kruskal-Wallis test, Kruskal-Wallis test with Dunn's multiple comparison, Spearman's rank correlation or an ensemble thereof; and calculating the Lupus Disease Activity Immune Index (LDAII), wherein the LDAII score distinguishes between active SLE and low SLE disease activity (low clinical disease (SLEDAI < 4). In another aspect, the method further comprises differentiating clinically and serologically quiescent (CQSQ) SLE patients compared to healthy controls. In another aspect, at least 7, 8, 9, 10, 11, 12. 13, 14, 15, 16, 17, 18, 19, 20, 21, 22, 23, 24, 25, 26, 27, 28, 29, 30, 31, 32, 33, 34, 35, 36, 37, 38, or 39 biomarkers are used in the calculation of the LDAII. In another aspect, the immunoassay is a multiplexed immunoassay. The LDAII is calculated as follows: a concentration biomarkers is determined and log-transformed for the subject and each log-transformed soluble mediator level determined for the subject sample is standardized as follows: (observed value)-(mean value of all SLE patients and healthy control visits)/(standard deviation of all SLE patient and healthy control visits); generating Spearman coefficients from a linear regression model testing associations between one or more auto-antibody (AutoAb) specificities for each soluble mediator assessed in the SLE patient compared to healthy controls (Spearman r); transforming and standardizing the values of the soluble mediator levels of the subject and the values weighted (multiplied) by their respective Spearman coefficients (Spearman r); and summing for each participant visit, the log transformed, standardized and weighted values for each of the four or more soluble mediators to calculate the LDAII. In another aspect, an increase in the LDAII is indicative of at least one of: SLE disease progression, increased autoimmune disease activity, or organ damage.

### BRIEF DESCRIPTION OF THE DRAWINGS

For a more complete understanding of the features and advantages of the present invention, reference is now made to the detailed description of the invention along with the accompanying figures and in which:

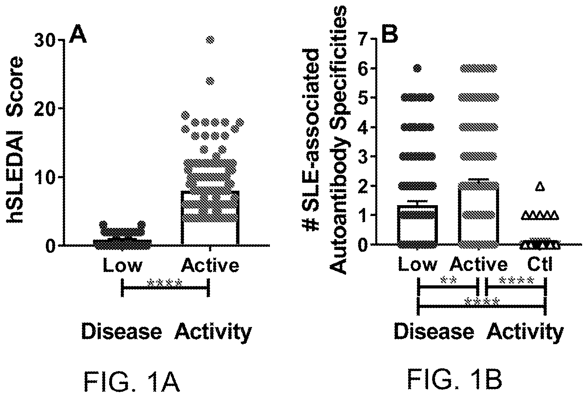

FIGS. 1A and 1B show increased hSLEDAI and number of AutoAb specificities in SLE patients with active disease. (FIG. 1A) Hybrid SLEDAI (SELENA-SLEDAI with proteinuria as defined by SLEDAI-2K) in SLE patient visits with low (hSLEDAI < 4; n=132) and active (hSLEDAI ≥ 4; n=179) disease; mean ± SEM, *p*<*0.0001* by unpaired t-test. (FIG. 1B) Number of SLE-associated AutoAb specificities (Abs to dsDNA, chromatin, Ro/SSA, La/SSB, Sm, SmRNP, and RNP) in SLE patients with low or active disease vs. matched healthy controls (Ctls; n=48). Levels are presented as the mean ±SEM. ***p*<*0.01*; *****p*<*0.0001* by Kruskal-Wallis test with Dunn's multiple comparison.

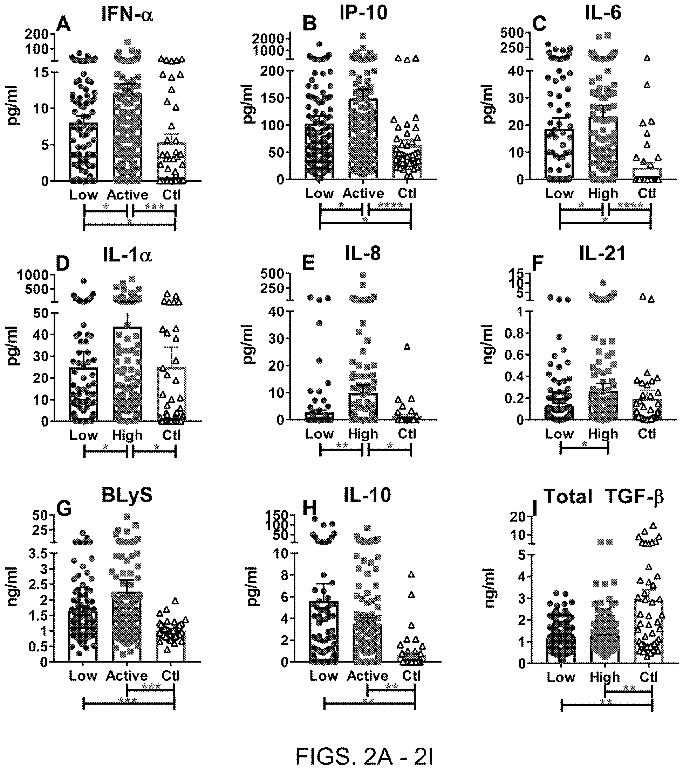

FIGS. 2A to 2I show altered soluble mediators in SLE low vs. active disease and Ctls. Plasma samples from SLE patients during visits with low (n=132) disease activity were compared to SLE patient samples procured during visits with active disease (n=179) vs. matched Ctls (n=48). Factors examined included: (FIG. 2A) IFN-α, (FIG. 2B) IP-10, (FIG. 2C) IL-6, (FIG. 2D) IL-la, (FIG. 2E) IL-8, (FIG. 2F) IL-21, (FIG. 2G) BLyS, (FIG. 2H) IL-10, and (FIG. 2I) total TGF-β. Levels are presented as the mean ± SEM. **p*<*0.05*; ***p*<*0.01*, ****p*<*0.001*, ****p*<*0.0001* by Kruskal-Wallis test with Dunn's multiple comparison.

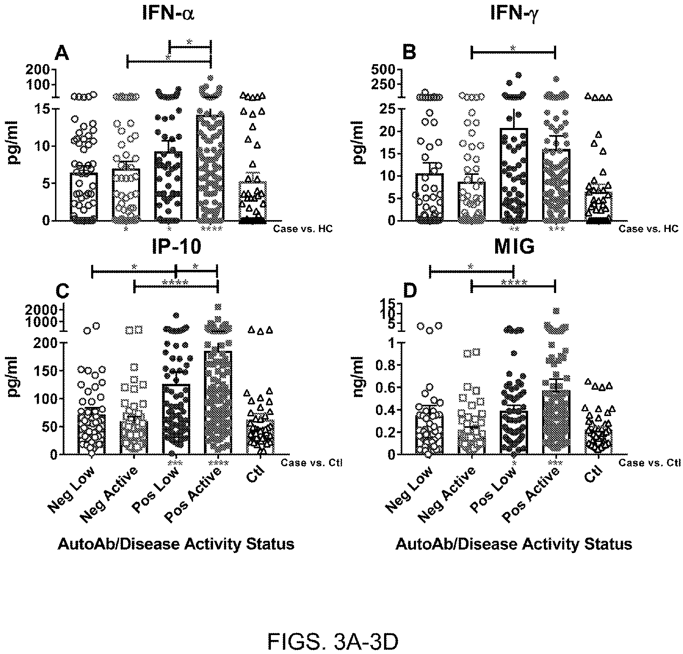

FIGS. 3A to 3D show IFN-associated mediators altered with disease activity/AutoAb positivity. SLE patient visits with AutoAb negative (Neg)/low disease activity (Low) samples (n=56) were compared to AutoAb Neg/Active disease (Active; n=52), AutoAb positive (Pos)/Low (n=76), and AutoAb Pos/Active samples. SLE patient samples were also compared to matched Ctl samples (n=48). Factors examined included: (FIG. 3A) IFN-α, (FIG. 3B) IFN-γ, (FIG. 3C) IP-10, and (FIG. 3D) MIG. Levels are presented as the mean ± SEM**p*<*0.05*; ***p*<*0.01*, ****p*<*0.001*, ****p*<*0.0001* by Kruskal-Wallis test with Dunn's multiple comparison. Cases (SLE patient groups) were compared to Ctls by Mann-Whitney test.

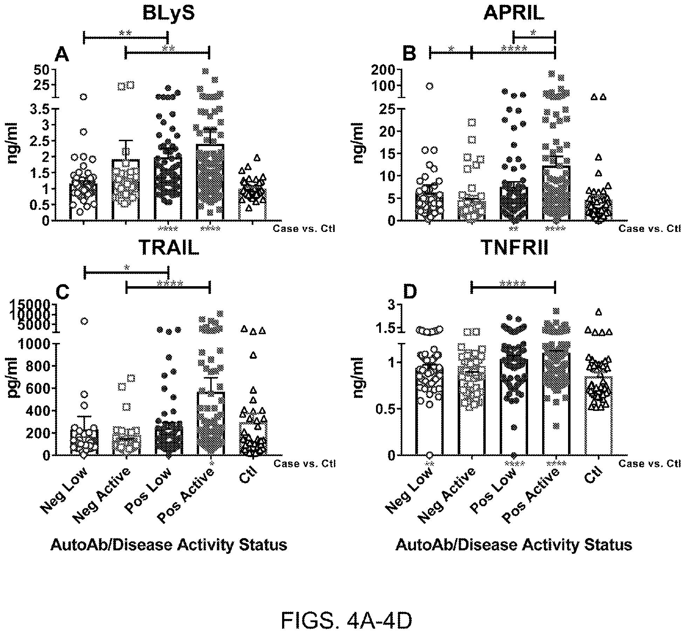

FIGS. 4A to 4D show TNF superfamily mediators altered with disease activity/AutoAb positivity. SLE patient visits with AutoAb negative (Neg)/low disease activity (Low) samples (n=56) were compared to AutoAb Neg/Active disease (Active; n=52), AutoAb positive (Pos)/Low (n=76), and AutoAb Pos/Active samples. SLE patient samples were also compared to matched Ctl samples (n=48). Factors examined included: (FIG. 4A) BLyS, (FIG. 4B) APRIL, (FIG. 4C) TRAIL, and (FIG. 4D) TNFRII. Levels are presented as the mean ± SEM. **p*<*0.05*; ***p*<*0.01*, *****p*<*0.0001* by Kruskal-Wallis test with Dunn's multiple comparison. Cases (SLE patient groups) were compared to Ctls by Mann-Whitney test.

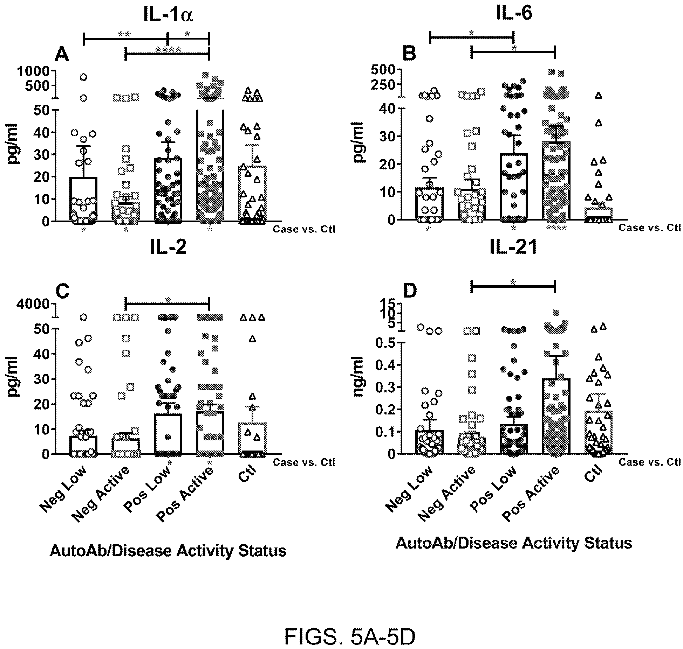

FIGS. 5A to 5D show innate and adaptive mediators altered with disease activity/AutoAb positivity. SLE patient visits with AutoAb negative (Neg)/low disease activity (Low) samples (n=56) were compared to AutoAb Neg/Active disease (Active; n=52), AutoAb positive (Pos)/Low (n=76), and AutoAb Pos/Active samples. SLE patient samples were also compared to matched Ctl samples (n=48). Factors examined included: (FIG. 5A) IL-1a, (FIG. 5B) IL-6, (FIG. 5C) IL-2, and (FIG. 5D) IL-21. Levels are presented as the mean ± SEM; **p*<*0.05*; ***p*<*0.01*, *****p*<*0.0001* by Kruskal-Wallis test with Dunn's multiple comparison. Cases (SLE patient groups) were compared to Ctls by Mann-Whitney test.

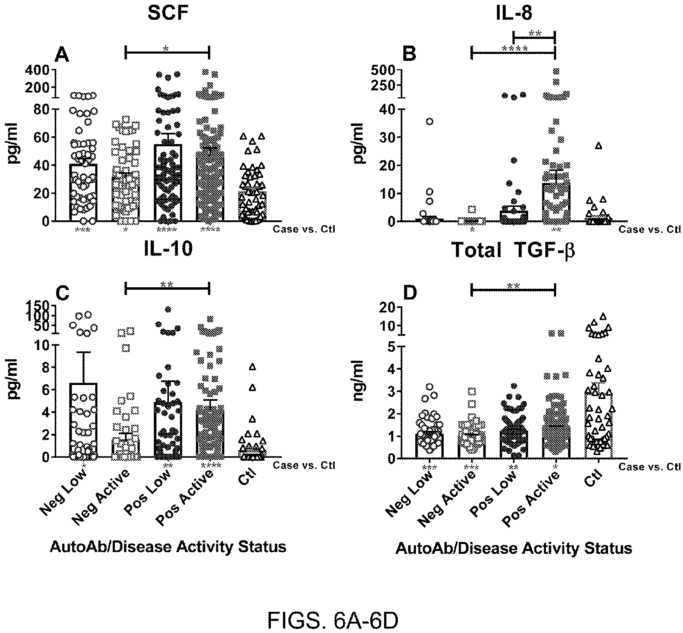

FIGS. 6A to 6D show inflammatory and regulatory mediators altered with disease activity/AutoAb positivity. SLE patient visits with AutoAb negative (Neg)/low disease activity (Low) samples (n=56) were compared to AutoAb Neg/Active disease (Active; n=52), AutoAb positive (Pos)/Low (n=76), and AutoAb Pos/Active samples. SLE patient samples were also compared to matched Ctl samples (n=48). Factors examined included: (FIG. 6A) SCF, (FIG. 6B) IL-8, (FIG. 6C) IL-10, and (FIG. 6D) total TGF-β. Levels are presented as the mean ± SEM**p*<*0.05*; ***p*<*0.01*, ****p*<*0.001*, ****p*<*0.0001* by Kruskal-Wallis test with Dunn's multiple comparison. Cases (SLE patient groups) were compared to Ctls by Mann-Whitney test.

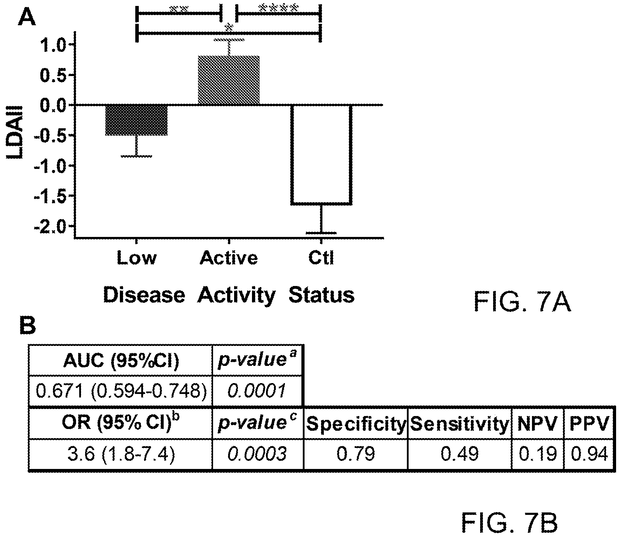

FIGS. 7A and 7B show Lupus Disease Activity Immune Index (LDAII) differentiates SLE patients with low and active disease vs. Ctls. (FIG. 7A) The LDAII was calculated for SLE patients with low or active disease vs. Ctls. LDAII is shown as mean ± SEM; **p*<*0.05*; ***p*<*0.01*, ****p*<*0.0001* by Kruskal-Wallis test with Dunn's multiple comparison. (FIG. 7B) Area under the curve (AUC)^{a} for differentiation SLE patients (low or active disease) from Ctls. Odds Ratio^{b} was calculated based on likelihood of having a positive LDAII score with active lupus; p-value^{c}, specificity, sensitivity, negative predictive value (NPV), and positive predictive value (PPV) were calculated by Fisher's exact test.

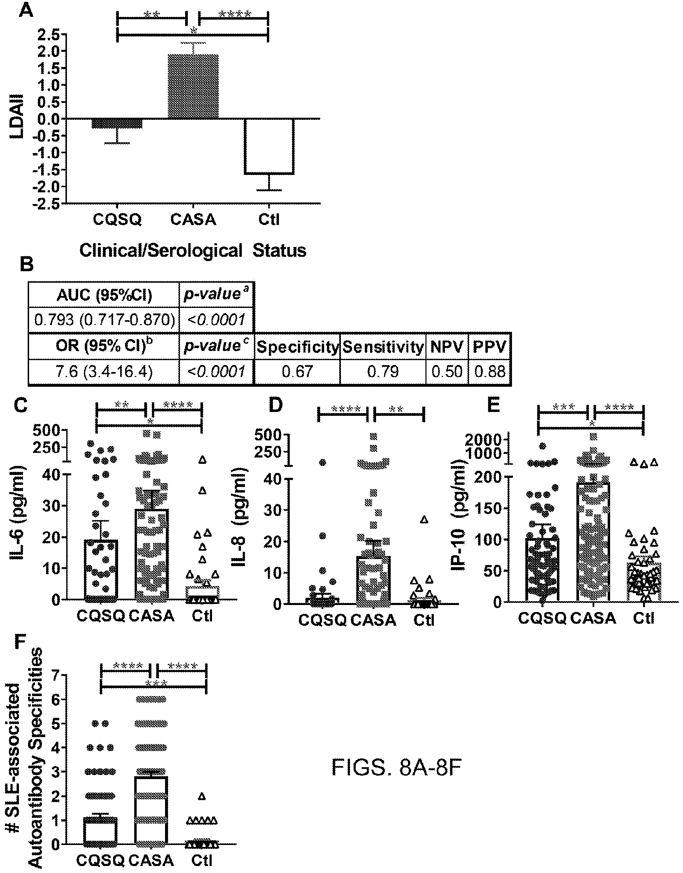

FIGS. 8A to 8F show Lupus Disease Activity Immune Index (LDAII) differentiates SLE patients with clinically and serologically active vs. quiescent disease vs. Ctls. (FIG. 8A) The LDAII was calculated for SLE patients with clinically and serologically quiescent (CQSQ; n=73) or clinically and serologically active (CASA; n=115) disease vs. Ctls. LDAII is shown as mean ± SEM; **p*<*0.05*; ***p<0.01,* ****p*<*0.0001* by Kruskal-Wallis test with Dunn's multiple comparison. (FIG. 8B) Area under the curve (AUC)^{a} for differentiation SLE patients (low or active disease) from Ctls. Odds Ratio^{b} was calculated based on likelihood of having a positive LDAII score with active lupus; p-value^{c}, specificity, sensitivity, negative predictive value (NPV), and positive predictive value (PPV) were calculated by Fisher's exact test. Plasma levels of distinct factors, including IL-6 (FIG. 8C), IL-8 (FIG. 8D), and IP-10 (FIG. 8E), as well as number of AutoAb specificities (FIG. 8F) were also determined. Levels are presented as the mean ± SEM; **p*<*0.05*; ***p*<*0.01*, ****p*<*0.001*, *****p*<*0.0001* by Kruskal-Wallis test with Dunn's multiple comparison.

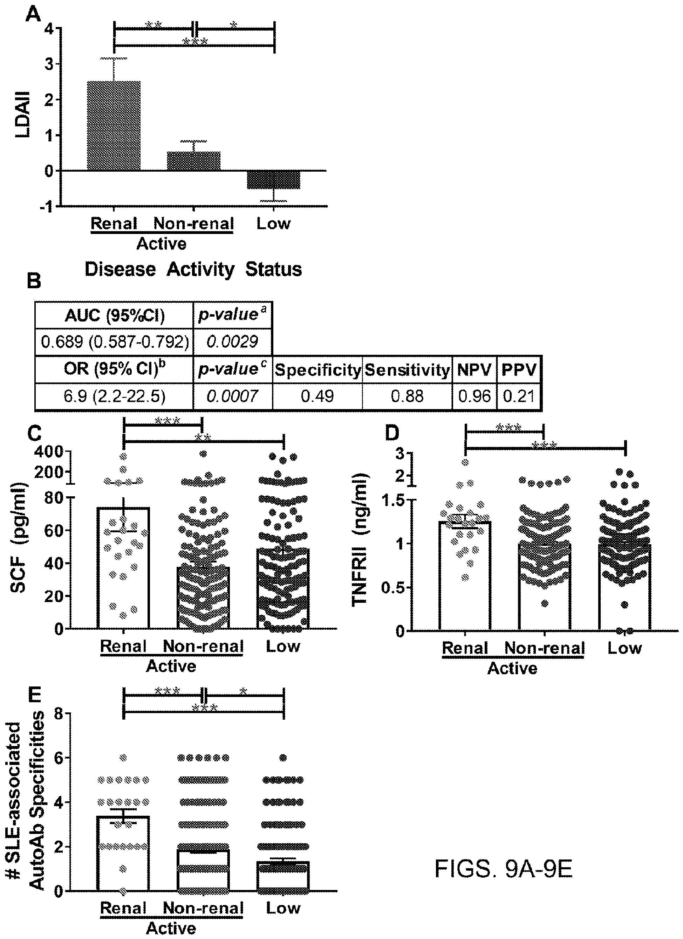

FIGS. 9A to 9E show Lupus Disease Activity Immune Index (LDAII) differentiates SLE patients with renal or non-renal, active disease compared to low disease activity. (FIG. 9A) The LDAII was calculated for SLE patient visits with renal (n=24) or non-renal (n=155) active disease vs. SLE patient visits with low disease activity (n=132). LDAII is shown as mean ± SEM; **p*<*0.05*; ***p*<*0.01*, ****p*<*0.0001* by Kruskal-Wallis test with Dunn's multiple comparison. (FIG. 9B) Area under the curve (AUC)^{a} for differentiation SLE patients with renal manifestations. Odds Ratio^{b} was calculated based on likelihood of having a positive LDAII score with active lupus and renal manifestations; p-value^{c}, specificity, sensitivity, negative predictive value (NPV), and positive predictive value (PPV) were calculated by Fisher's exact test. Plasma levels of distinct factors, including SCF (FIG. 9C), TNFRII (FIG. 9D), and number of AutoAb specificities (FIG. 9E) were also determined. Levels are presented as the mean ± SEM; **p*<*0.05*; ***p*<*0.01*, ****p*<*0.001* by Kruskal-Wallis test with Dunn's multiple comparison.

### DETAILED DESCRIPTION OF THE INVENTION

While the making and using of various embodiments of the present invention are discussed in detail below, it should be appreciated that the present invention provides many applicable inventive concepts that can be embodied in a wide variety of specific contexts. The specific embodiments discussed herein are merely illustrative of specific ways to make and use the invention and do not delimit the scope of the invention, which is set out in the appended claims.

To facilitate the understanding of this invention, a number of terms are defined below. Terms defined herein have meanings as commonly understood by a person of ordinary skill in the areas relevant to the present invention. Terms such as "a", "an" and "the" are not intended to refer to only a singular entity, but include the general class of which a specific example may be used for illustration. The terminology herein is used to describe specific embodiments of the invention, but their usage does not limit the invention, except as outlined in the claims.

The present inventors leveraged plasma samples serially collected from Systemic lupus erythematosus (SLE) patients in the Oklahoma Lupus Cohort to compare levels and determine temporal relationships between autoantibodies and immune mediators from multiple immune pathways in SLE patients with low or active disease compared to matched, healthy controls. The present invention sheds light on potential mechanisms of immunopathogenesis as it relates to clinical disease activity, whereby dysregulation of immune mediators occurs alongside and independent of autoantibody accumulation. Further, the present invention includes the design and validation of a reliable and sensitive tool to assess the immune status of lupus patients as it relates to clinical disease activity. The present invention can be used to identify high risk patients in need of rheumatology referral and enrollment in prospective, preclinical intervention studies, as well as inform the development of novel treatment strategies to avert or delay tissue damage.

As used herein, the term "dataset" refers to a set of numerical values resulting from evaluation of a sample (or population of samples) under a desired condition. The values of the dataset can be obtained, for example, by experimentally obtaining measures from a sample, such as a patient sample, and building a dataset from these measurements. Alternatively, the dataset can be obtained from a database or a server on which the dataset has been stored, or even a service provider such as an internal or third party laboratory.

As used herein, the term "disease" in the context of the present invention refers to any disorder, condition, sickness, or ailment, that manifests in, for example, a dysfunctional or incorrectly functioning immune system that causes, e.g., SLE.

As used herein, the term "sample" refers to any biological sample that is isolated from a subject, which can include, without limitation, a single cell or multiple cells, fragments of cells, an aliquot of body fluid, whole blood, platelets, serum, plasma, red blood cells, white blood cells or leucocytes, endothelial cells, tissue biopsies, synovial fluid, lymphatic fluid, ascites fluid, and interstitial or extracellular fluid. The term "sample" also encompasses the fluid in spaces between cells, including gingival crevicular fluid, bone marrow, cerebrospinal fluid, saliva, mucous, sputum, semen, sweat, urine, or any other bodily fluids.

As used herein, the term "blood sample" refers to whole blood or any fraction thereof, including blood cells, red blood cells, white blood cells or leucocytes, platelets, serum and plasma. Samples can be obtained from a subject by means including but not limited to venipuncture, excretion, ejaculation, massage, biopsy, needle aspirate, lavage, scraping, surgical incision, or intervention or other means known in the art.

As used herein, the term "subject" or "patient" refers generally to a mammal, which includes, but is not limited to, a human, non-human primate, dog, cat, mouse, rat, cow, horse, and pig, without regard to gender or age. A subject can be one who has been previously diagnosed or identified as having an auto-immune and/or inflammatory disease, and which may have already undergone, or is undergoing, a therapeutic intervention for the auto-immune and/or inflammatory disease. However, a subject can also include a patient not previously diagnosed as having the auto-immune and/or inflammatory disease, for example, a subject who exhibits one or more symptoms or risk factors for the auto-immune and/or inflammatory disease, or a subject who does not exhibit symptoms or risk factors for the auto-immune and/or inflammatory disease, or a subject who is asymptomatic for the auto-immune and/or inflammatory disease.

As used herein, the phrase "innate serum or plasma mediator biomarker(s)" refers to one or more of the following biomarkers: IL-la, IL-1β, IL-1RA, IFN-α, IL-15, IL-12p70, IL-6, and IL-23p19. These biomarkers can be measured at the RNA or protein level and can be obtained from samples, e.g., blood, serum, plasma and/or urine sample from the patient, which is a mammal, e.g., a human patient. The abbreviations for all the biomarkers used herein are well-known to the skilled artisan, e.g., IL-1 is interleukin-1, and so forth. The abbreviations can be matched to the protein at, e.g., www.genecards.org.

As used herein, the phrase "adaptive serum or plasma mediator biomarker(s)" refers to one or more of the following biomarkers: IL-2, IFN-γ, IL-4, IL-5, IL-13, IL-17A, IL-21, IL-10, and TGF-β. These biomarkers can be measured at the RNA or protein level and can be obtained from samples, e.g., blood, serum, plasma and/or urine sample from the patient, which is a mammal, e.g., a human patient.

As used herein, the phrase "chemokine biomarker(s)" refers to one or more of the following biomarkers: IL-8/CXCL8, IP-10/CXCL10, MIG/CXCL9, MIP-1α/CCL3, MIP-1β/CCL4, MCP-1/CCL2, and MCP-3/CCL7. These biomarkers can be measured from samples, e.g., blood, serum, plasma and/or urine sample from the patient, which is a mammal, e.g., a human patient.

As used herein, the phrase "soluble TNF superfamily biomarker(s)" refers to one or more of the following biomarkers: TNF-α, TNFRI, TNFRII, Fas, CD40L/CD154, BLyS, and APRIL or tumor necrosis factor ligand superfamily member 13 (TNFSF13). These biomarkers can be measured at the RNA or protein level and can be obtained from samples, e.g., blood, serum, plasma and/or urine sample from the patient, which is a mammal, e.g., a human patient.

As used herein, the phrase "inflammatory mediator biomarker(s)" refers to one or more of the following biomarkers: Stem Cell Factor (SCF), Plasminogen Activator Inhibitor 1 (PAI-1), and Resistin. These biomarkers can be measured at the RNA or protein level and can be obtained from samples, e.g., blood, serum, plasma and/or urine sample from the patient, which is a mammal, e.g., a human patient.

As used herein, the phrase "SLE-associated autoantibody specificity biomarker(s)" refers to one or more of the following biomarkers that are autoantibodies against the following targets: dsDNA, chromatin, RiboP, Ro/SSA, La/SSB, Sm, SmRNP, and RNP, all of which are well-known to the skilled artisan in the SLE arts. These biomarkers can be measured at the RNA or protein level and can be obtained from samples, e.g., blood, serum, plasma and/or urine sample from the patient, which is a mammal, e.g., a human patient.

As used herein, a "healthy control" refers to a healthy control that is not an SLE patient that has no clinical evidence of SLE.

Described herein are methods for identifying and changing the treatment of SLE patients associated with clinical disease activity as defined by the Safety of Estrogens in Lupus National Assessment-Systemic Lupus Erythematosus Disease Activity Index (SELENA-SLEDAI) with proteinuria as defined by the SLEDAI-2K, known as the hybrid-SLEDAI (hSLEDAI). As clinical manifestations of SLE contribute to disease activity in the hSLEDAI after they have occurred, the present invention is used to determine if the subject may be exhibiting biomarkers that may contribute to disease activity that places the SLE patient at risk for permanent organ damage and early mortality.

Following the ACR criteria for SLE classification, patients must meet at least 4 ACR criteria for SLE to reach disease classification (diagnosis), including: malar rash, discoid rash, photosensitivity, oral ulcers, arthritis, serositis (pleuritis or pericarditis), renal disorder (proteinuria or cellular casts), neurologic disorder (seizures or psychosis), hematologic disorder (hemolytic anemia, leukopenia, lymphopenia, or thrombocytopenia), immunologic disorder (anti-DNA, anti-Sm, or anti-phospholipid antibodies ), and positive ANA (HEp-2 IIF assay). Other criteria may also be used, as known to the skilled artisan, e.g., using the Systemic Lupus International Collaborating Clinics (SLICC) rule for the classification of SLE, the patient must satisfy at least 4 criteria, including at least one clinical criterion and one immunologic criterion OR the patient must have biopsy proven lupus nephritis in the presence of antinuclear antibodies or anti-double-stranded DNA antibodies.

Biomarker detection. There are a variety of methods that can be used to assess protein expression. One such approach is to perform protein identification with the use of antibodies.

As used herein, the term "antibody" refers, broadly, to any immunologic binding agent such as IgG, IgM, IgA, IgD and IgE antibody, or subclass thereof, or binding fragments thereof, including single chain fragments. Generally, IgG and/or IgM are used because they are the most common antibodies in the physiological situation and because are commonly and easily made in a laboratory setting. As used herein, the term "antibody fragment" refers to any antibody-like molecule that has an antigen binding region, and includes antibody fragments such as Fab', Fab, F(ab')₂, single domain antibodies (DABs), Fv, scFv (single chain Fv), and the like. The techniques for preparing and using various antibody-based constructs and fragments are well known in the art. Means for preparing and characterizing antibodies, both polyclonal and monoclonal, are also well known in the art (see, e.g., Antibodies: A Laboratory Manual, Cold Spring Harbor Laboratory, 1988).

In accordance with the present invention, examples of immunodetection methods are provided. Some immunodetection methods include enzyme-linked immunosorbent assay (ELISA), radioimmunoassay (RIA), immunoradiometric assay, fluoroimmunoassay, chemiluminescent assay, bioluminescent assay, and Western blot to mention a few. The steps of various useful immunodetection methods have been described in the scientific literature, Current Protocols in Immunology, Wiley & Sons Press, 2017.

In general, the immunobinding methods include obtaining a sample suspected of containing a relevant polypeptide, and contacting the sample with a first antibody under conditions effective to allow the formation of immunocomplexes. In terms of antigen detection, the biological sample analyzed may be any sample that is suspected of containing an antigen, such as, for example, a tissue section or specimen, a homogenized tissue extract, a cell, or even a biological fluid.

Contacting the chosen biological sample with the antibody under effective conditions and for a period of time sufficient to allow the formation of immune complexes (primary immune complexes) is generally a matter of simply adding the antibody composition to the sample and incubating the mixture for a period of time long enough for the antibodies to form immune complexes with, i.e., to bind to, any antigens present. After this time, the sample-antibody composition, such as a tissue section, ELISA plate, dot blot or western blot, will generally be washed to remove any non-specifically bound antibody species, allowing only those antibodies specifically bound within the primary immune complexes to be detected.

In general, the detection of immunocomplex formation is well known in the art and may be achieved through the application of numerous approaches. These methods are generally based upon the detection of a label or marker, such as any of those radioactive, fluorescent, biological and enzymatic tags. Patents concerning the use of such labels include U.S. Pat. Nos. 3,817,837; 3,850,752; 3,939,350; 3,996,345; 4,277,437; 4,275,149 and 4,366,241. Of course, one may find additional advantages through the use of a secondary binding ligand such as a second antibody and/or a biotin/avidin ligand binding arrangement, as is known in the art.

The antibody, or binding fragment thereof, can be employed in the detection may itself be linked to a detectable label, wherein one would then simply detect this label, thereby allowing the amount of the primary immune complexes in the composition to be determined. Alternatively, the first antibody that becomes bound within the primary immune complexes may be detected by means of a second binding ligand that has binding affinity for the antibody. In these cases, the second binding ligand may be linked to a detectable label. The second binding ligand is itself often an antibody, which may thus be termed a "secondary" antibody. The primary immune complexes are contacted with the labeled, secondary binding ligand, or antibody, under effective conditions and for a period of time sufficient to allow the formation of secondary immune complexes. The secondary immune complexes are then generally washed to remove any non-specifically bound labeled secondary antibodies or ligands, and the remaining label in the secondary immune complexes is then detected.

Further methods include the detection of primary immune complexes by a two-step approach. A second binding ligand, such as an antibody, that has binding affinity for the antibody is used to form secondary immune complexes, as described above. After washing, the secondary immune complexes are contacted with a third binding ligand or antibody that has binding affinity for the second antibody, again under effective conditions and for a period of time sufficient to allow the formation of immune complexes (tertiary immune complexes). The third ligand or antibody is linked to a detectable label, allowing detection of the tertiary immune complexes thus formed. This system may provide for signal amplification if this is desired.

One method of immunodetection uses two different antibodies. A first step biotinylated, monoclonal or polyclonal antibody is used to detect the target antigen(s), and a second step antibody is then used to detect the biotin attached to the complexed biotin. In that method the sample to be tested is first incubated in a solution containing the first step antibody. If the target antigen is present, some of the antibody binds to the antigen to form a biotinylated antibody/antigen complex. The antibody/antigen complex is then amplified by incubation in successive solutions of streptavidin (or avidin), biotinylated DNA, and/or complementary biotinylated DNA, with each step adding additional biotin sites to the antibody/antigen complex. The amplification steps are repeated until a suitable level of amplification is achieved, at which point the sample is incubated in a solution containing the second step antibody against biotin. This second step antibody is labeled, as for example with an enzyme that can be used to detect the presence of the antibody/antigen complex by histoenzymology using a chromogen substrate. With suitable amplification, a conjugate can be produced which is macroscopically visible.

Another known method of immunodetection takes advantage of the immuno-PCR (Polymerase Chain Reaction) methodology. The PCR method is similar to the Cantor method up to the incubation with biotinylated DNA, however, instead of using multiple rounds of streptavidin and biotinylated DNA incubation, the DNA/biotin/streptavidin/antibody complex is washed out with a low pH or high salt buffer that releases the antibody. The resulting wash solution is then used to carry out a PCR reaction with suitable primers with appropriate controls. At least in theory, the enormous amplification capability and specificity of PCR can be utilized to detect a single antigen molecule.

As detailed above, immunoassays are in essence binding assays. Certain immunoassays are the various types of ELISAs and RIA known in the art. However, it will be readily appreciated that detection is not limited to such techniques, and Western blotting, dot blotting, FACS analyses, and the like may also be used.

In one exemplary ELISA, the antibodies are immobilized onto a selected surface exhibiting protein affinity, such as a well in a polystyrene microtiter plate. Then, a test composition suspected of containing the antigen, such as a clinical sample, is added to the wells. After binding and washing to remove non-specifically bound immune complexes, the bound antigen may be detected. Detection is generally achieved by the addition of another antibody that is linked to a detectable label. This type of ELISA is a simple "sandwich ELISA." Detection may also be achieved by the addition of a second antibody, followed by the addition of a third antibody that has binding affinity for the second antibody, with the third antibody being linked to a detectable label.

In another exemplary ELISA, the samples suspected of containing the antigen are immobilized onto the well surface and then contacted with the anti-ORF message and anti-ORF translated product antibodies. After binding and washing to remove non-specifically bound immune complexes, the bound anti-ORF message and anti-ORF translated product antibodies are detected. Where the initial anti-ORF message and anti-ORF translated product antibodies are linked to a detectable label, the immune complexes may be detected directly. Again, the immune complexes may be detected using a second antibody that has binding affinity for the first anti-ORF message and anti-ORF translated product antibody, with the second antibody being linked to a detectable label.

Another type of ELISA in which the antigens are immobilized, involves the use of antibody competition in the detection. In this ELISA, labeled antibodies against an antigen are added to the wells, allowed to bind, and detected by means of their label. The amount of an antigen in an unknown sample is then determined by mixing the sample with the labeled antibodies against the antigen during incubation with coated wells. The presence of an antigen in the sample acts to reduce the amount of antibody against the antigen available for binding to the well and thus reduces the ultimate signal. This is also appropriate for detecting antibodies against an antigen in an unknown sample, where the unlabeled antibodies bind to the antigen-coated wells and also reduces the amount of antigen available to bind the labeled antibodies.

As used herein, the phrase "under conditions effective to allow immune complex (antigen/antibody) formation" refers to those conditions, which may also include diluting the antigens and/or antibodies with solutions such as BSA, bovine gamma globulin (BGG) or phosphate buffered saline (PBS)/Tween, under which an antibody or binding fragment thereof interacts with the antigen that is the specific target of the antibody. These added agents also tend to assist in the reduction of nonspecific background. The "suitable" conditions such that the incubation is at a temperature or for a period of time sufficient to allow effective binding. Incubation steps are typically from about 1 to 2 to 4 hours or so, at temperatures preferably on the order of 25°C. to 27°C., or may be overnight at about 4°C. or so.

Another antibody-based approach to assessing biomarkers expression is Fluorescence-Activated Cell Sorting (FACS), a specialized type of flow cytometry. It provides a method for sorting a heterogeneous mixture of biological cells into two or more containers, one cell at a time, based upon the specific light scattering and fluorescent characteristics of each cell. It provides fast, objective and quantitative recording of fluorescent signals from individual cells as well as physical separation of cells of particular interest. A cell suspension is entrained in the center of a narrow, rapidly flowing stream of liquid. The flow is arranged so that there is a large separation between cells relative to their diameter. A vibrating mechanism causes the stream of cells to break into individual droplets. The system is adjusted so that there is a low probability of more than one cell per droplet. Just before the stream breaks into droplets, the flow passes through a fluorescence measuring station where the fluorescent character of interest of each cell is measured. An electrical charging ring is placed just at the point where the stream breaks into droplets. A charge is placed on the ring based on the immediately prior fluorescence intensity measurement, and the opposite charge is trapped on the droplet as it breaks from the stream. The charged droplets then fall through an electrostatic deflection system that diverts droplets into containers based upon their charge. In some systems, the charge is applied directly to the stream, and the droplet breaking off retains charge of the same sign as the stream. The stream is then returned to neutral after the droplet breaks off. One common way to use FACS is with a fluorescently labeled antibody that binds to a target on or in a cell, thereby identifying cells with a given target. This technique can be used quantitatively where the amount of fluorescent activity correlates to the amount of target, thereby permitting one to sort based on relative amounts of fluorescence, and hence relative amounts of the target.

Bead-based xMAP Technology may also be applied to immunologic detection in conjunction with the presently claimed invention. This technology combines advanced fluidics, optics, and digital signal processing with proprietary microsphere technology to deliver multiplexed assay capabilities. Featuring a flexible, open-architecture design, xMAP technology can be configured to perform a wide variety of bioassays quickly, cost-effectively and accurately.

Fluorescently-coded microspheres are arranged in up to 500 distinct sets. Each bead set can be coated with a reagent specific to a particular bioassay (e.g., an antibody), allowing the capture and detection of specific analytes from a sample, such as the biomarkers of the present application. Inside the xMAP multiplex analyzer, a light source excites the internal dyes that identify each microsphere particle, and also any reporter dye captured during the assay. Many readings are made on each bead set, which further validates the results. Using this process, xMAP technology allows multiplexing of up to 500 unique bioassays within a single sample, both rapidly and precisely. Unlike other flow cytometer microsphere-based assays which use a combination of different sizes and color intensities to identify an individual microsphere, xMAP technology uses 5.6 micron size microspheres internally dyed with red and infrared fluorophores via a proprietary dying process to create 500 unique dye mixtures which are used to identify each individual microsphere.

Some of the advantages of xMAP include multiplexing (reduces costs and labor), generation of more data with less sample, less labor and lower costs, faster, more reproducible results than solid, planar arrays, and focused, flexible multiplexing of 1 to 500 analytes to meet a wide variety of applications.

Nucleic Acid Detection. In other embodiments for detecting protein expression, one may assay for gene transcription. For example, an indirect method for detecting protein expression is to detect mRNA transcripts from which the proteins are made.

Amplification of Nucleic Acids. Since many mRNAs are present in relatively low abundance, nucleic acid amplification greatly enhances the ability to assess expression. The general concept is that nucleic acids can be amplified using paired primers flanking the region of interest. As used herein, the term "primer," refers to any nucleic acid that is capable of priming the synthesis of a nascent nucleic acid in a template-dependent process. Typically, primers are oligonucleotides from ten to twenty and/or thirty base pairs in length, but longer sequences can be employed. Primers may be provided in double-stranded and/or single-stranded form, although the single-stranded form is often used.

Pairs of primers designed to selectively hybridize to nucleic acids corresponding to selected genes are contacted with the template nucleic acid under conditions that permit selective hybridization. Depending upon the desired application, high stringency hybridization conditions may be selected that will only allow hybridization to sequences that are completely complementary to the primers. In other embodiments, hybridization may occur under reduced stringency to allow for amplification of nucleic acids containing one or more mismatches with the primer sequences. Once hybridized, the template-primer complex is contacted with one or more enzymes that facilitate template-dependent nucleic acid synthesis. Multiple rounds of amplification, also referred to as "cycles," are conducted until a sufficient amount of amplification product is produced.

The amplification product may be detected or quantified. In certain applications, the detection may be performed by visual method. Alternatively, the detection may involve indirect identification of the product via chemilluminescence, radioactive scintigraphy of incorporated radiolabel or fluorescent label or even via a system using electrical and/or thermal impulse signals.

A number of template dependent processes are available to amplify the oligonucleotide sequences present in a given template sample. One of the best known amplification methods is the polymerase chain reaction (PCR) which is described in detail in U.S. Pat. Nos. 4,683,195, 4,683,202 and 4,800,159.

A reverse transcriptase-PCR amplification procedure may be performed to quantify the amount of mRNA amplified. Methods of reverse transcribing RNA into cDNA are well known (see Sambrook et al., Molecular Cloning: A Laboratory Manual, 2001). Alternative methods for reverse transcription utilize thermostable DNA polymerases. These methods are described in WO 90/07641. Polymerase chain reaction methodologies are well known in the art. Representative methods of RT-PCR are described in U.S. Pat. No. 5,882,864. Standard PCR usually uses one pair of primers to amplify a specific sequence, while multiplex-PCR (MPCR) uses multiple pairs of primers to amplify many sequences simultaneously. The presence of many PCR primers in a single tube could cause many problems, such as the increased formation of misprimed PCR products and "primer dimers", the amplification discrimination of longer DNA fragment and so on. Normally, MPCR buffers contain a Taq Polymerase additive, which decreases the competition among amplicons and the amplification discrimination of longer DNA fragment during MPCR. MPCR products can further be hybridized with gene-specific probe for verification. Theoretically, one should be able to use as many as primers as necessary. However, due to side effects (primer dimers, misprimed PCR products, etc.) caused during MPCR, there is a limit (less than 20) to the number of primers that can be used in a MPCR reaction. See also European Application No. 0 364 25 5.

Another method for amplification is ligase chain reaction ("LCR"), disclosed in European Application No. 320 308, incorporated herein by reference in its entirety. U.S. Pat. No. 4,883,750 describes a method similar to LCR for binding probe pairs to a target sequence. A method based on PCR and oligonucleotide ligase assay (OLA), disclosed in U.S. Pat. No. 5,912,148, may also be used. Alternative methods for amplification of target nucleic acid sequences that may be used in the practice of the present invention are disclosed in U.S. Pat. Nos. 5,843,650, 5,846,709, 5,846,783, 5,849,546, 5,849,497, 5,849,547, 5,858,652, 5,866,366, 5,916,776, 5,922,574, 5,928,905, 5,928,906, 5,932,451, 5,935,825, 5,939,291 and 5,942,391, GB Application No. 2 202 328, and in PCT Application No. PCT/US89/01025.

Detection of Nucleic Acids. Following any amplification, it may be desirable to separate the amplification product from the template and/or the excess primer. In one embodiment, amplification products are separated by agarose, agarose-acrylamide or polyacrylamide gel electrophoresis using standard methods (Sambrook et al., Molecular Cloning: A Laboratory Manual, 2001). Separated amplification products may be cut out and eluted from the gel for further manipulation. Using low melting point agarose gels, the separated band may be removed by heating the gel, followed by extraction of the nucleic acid. Separation of nucleic acids may also be effected by chromatographic techniques known in art. There are many kinds of chromatography which may be used in the practice of the present invention, including adsorption, partition, ion-exchange, hydroxylapatite, molecular sieve, reverse-phase, column, paper, thin-layer, and gas chromatography as well as HPLC.

In certain embodiments, the amplification products are visualized. A typical visualization method involves staining of a gel with ethidium bromide and visualization of bands under UV light. Alternatively, if the amplification products are integrally labeled with radio- or fluorometrically-labeled nucleotides, the separated amplification products can be exposed to x-ray film or visualized under the appropriate excitatory spectra.

In one embodiment, following separation of amplification products, a labeled nucleic acid probe is brought into contact with the amplified marker sequence. The probe preferably is conjugated to a chromophore but may be radiolabeled. In another embodiment, the probe is conjugated to a binding partner, such as an antibody or biotin, or another binding partner carrying a detectable moiety.

In particular embodiments, detection is by Southern blotting and hybridization with a labeled probe. The techniques involved in Southern blotting are well known to those of skill in the art (see Sambrook et al., Molecular Cloning: A Laboratory Manual, 2001). One example of the foregoing is described in U.S. Pat. No. 5,279,721, discloses an apparatus and method for the automated electrophoresis and transfer of nucleic acids. The apparatus permits electrophoresis and blotting without external manipulation of the gel and is ideally suited to carrying out methods according to the present invention.

Other methods of nucleic acid detection that may be used in the practice of the instant invention are disclosed in U.S. Pat. Nos. 5,840,873, 5,843,640, 5,843,651, 5,846,708, 5,846,717, 5,846,726, 5,846,729, 5,849,487, 5,853,990, 5,853,992, 5,853,993, 5,856,092, 5,861,244, 5,863,732, 5,863,753, 5,866,331, 5,905,024, 5,910,407, 5,912,124, 5,912,145, 5,919,630, 5,925,517, 5,928,862, 5,928,869, 5,929,227, 5,932,413 and 5,935,791.

Nucleic Acid Arrays. Microarrays include a plurality of polymeric molecules spatially distributed over, and stably associated with, the surface of a substantially planar substrate, e.g., biochips. Microarrays of polynucleotides have been developed and find use in a variety of applications, such as screening and DNA sequencing. One area in particular in which microarrays find use is in gene expression analysis.

In gene expression analysis with microarrays, an array of "probe" oligonucleotides is contacted with a nucleic acid sample of interest, i.e., target, such as polyA mRNA from a particular tissue type. Contact is carried out under hybridization conditions and unbound nucleic acid is then removed. The resultant pattern of hybridized nucleic acid provides information regarding the genetic profile of the sample tested. Methodologies of gene expression analysis on microarrays are capable of providing both qualitative and quantitative information.

A variety of different arrays that may be used with the present invention are known in the art. The probe molecules of the arrays which are capable of sequence specific hybridization with target nucleic acid may be polynucleotides or hybridizing analogues or mimetics thereof, including: nucleic acids in which the phosphodiester linkage has been replaced with a substitute linkage, such as phophorothioate, methylimino, methylphosphonate, phosphoramidate, guanidine and the like; nucleic acids in which the ribose subunit has been substituted, e.g., hexose phosphodiester; peptide nucleic acids; and the like. The length of the probes will generally range from 10 to 1,000 nucleotides, where in some embodiments the probes will be oligonucleotides and usually range from 15 to 150 nucleotides and more usually from 15 to 100 nucleotides in length, and in other embodiments the probes will be longer, usually ranging in length from 150 to 1,000 nucleotides, where the polynucleotide probes may be single- or double-stranded, usually single-stranded, and may be PCR fragments amplified from cDNA.

The probe molecules on the surface of the substrates will correspond to selected genes being analyzed and be positioned on the array at a known location so that positive hybridization events may be correlated to expression of a particular gene in the physiological source from which the target nucleic acid sample is derived. The substrates with which the probe molecules are stably associated may be fabricated from a variety of materials, including plastics, ceramics, metals, gels, membranes, glasses, and the like. The arrays may be produced according to any convenient methodology, such as preforming the probes and then stably associating them with the surface of the support or growing the probes directly on the support. A number of different array configurations and methods for their production are known to those of skill in the art and disclosed in U.S. Pat. Nos. 5,445,934, 5,532,128, 5,556,752, 5,242,974, 5,384,261, 5,405,783, 5,412,087, 5,424,186, 5,429,807, 5,436,327, 5,472,672, 5,527,681, 5,529,756, 5,545,531, 5,554,501, 5,561,071, 5,571,639, 5,593,839, 5,599,695, 5,624,711, 5,658,734, 5,700,637, and 6,004,755.

Following hybridization, where non-hybridized labeled nucleic acid is capable of emitting a signal during the detection step, a washing step is employed where unhybridized labeled nucleic acid is removed from the support surface, generating a pattern of hybridized nucleic acid on the substrate surface. A variety of wash solutions and protocols for their use are known to those of skill in the art and may be used. Where the label on the target nucleic acid is not directly detectable, one then contacts the array, now comprising bound target, with the other member(s) of the signal producing system that is being employed. For example, where the label on the target is biotin, one then contacts the array with streptavidin-fluorescent conjugate under conditions sufficient for binding between the specific binding member pairs to occur. Following contact, any unbound members of the signal producing system will then be removed, e.g., by washing. The specific wash conditions employed will necessarily depend on the specific nature of the signal producing system that is employed, and will be known to those of skill in the art familiar with the particular signal producing system employed. The resultant hybridization pattern(s) of labeled nucleic acids may be visualized or detected in a variety of ways, with the particular manner of detection being chosen based on the particular label of the nucleic acid, where representative detection means include scintillation counting, autoradiography, fluorescence measurement, calorimetric measurement, light emission measurement and the like.

Prior to detection or visualization, where one desires to reduce the potential for a mismatch hybridization event to generate a false positive signal on the pattern, the array of hybridized target/probe complexes may be treated with an endonuclease under conditions sufficient such that the endonuclease degrades single stranded, but not double stranded DNA. A variety of different endonucleases are known and may be used, where such nucleases include: mung bean nuclease, S1 nuclease, and the like. Where such treatment is employed in an assay in which the target nucleic acids are not labeled with a directly detectable label, e.g., in an assay with biotinylated target nucleic acids, the endonuclease treatment will generally be performed prior to contact of the array with the other member(s) of the signal producing system, e.g., fluorescent-streptavidin conjugate. Endonuclease treatment, as described above, ensures that only end-labeled target/probe complexes having a substantially complete hybridization at the 3' end of the probe are detected in the hybridization pattern. Following hybridization and any washing step(s) and/or subsequent treatments, as described above, the resultant hybridization pattern is detected. In detecting or visualizing the hybridization pattern, the intensity or signal value of the label will be not only be detected but quantified, by which is meant that the signal from each spot of the hybridization will be measured and compared to a unit value corresponding the signal emitted by known number of end-labeled target nucleic acids to obtain a count or absolute value of the copy number of each end-labeled target that is hybridized to a particular spot on the array in the hybridization pattern.

RNA Sequencing. RNA-seq (RNA Sequencing), also called Whole Transcriptome Shotgun Sequencing (WTSS), is a technology that utilizes the capabilities of Next-Generation Sequencing (NGS) to reveal a snapshot of RNA presence and quantity from a genome at a given moment in time. The transcriptome of a cell is dynamic; it continually changes as opposed to a static genome. The recent developments of next-generation sequencing allow for increased base coverage of a DNA sequence, as well as higher sample throughput. This facilitates sequencing of the RNA transcripts in a cell, providing the ability to look at alternative gene spliced transcripts, post-transcriptional changes, gene fusion, mutations/SNPs and changes in gene expression. In addition to mRNA transcripts, RNA-Seq can look at different populations of RNA to include total RNA, small RNA, such as miRNA, tRNA, and ribosomal profiling. RNA-Seq can also be used to determine exon/intron boundaries and verify or amend previously annotated 5' and 3' gene boundaries, Ongoing RNA-Seq research includes observing cellular pathway alterations during infection, and gene expression level changes in cancer studies. Prior to NGS, transcriptomics and gene expression studies were previously done with expression microarrays, which contain thousands of DNA sequences that probe for a match in the target sequence, making available a profile of all transcripts being expressed. This was later done with Serial Analysis of Gene Expression (SAGE).

Treatments for SLE. Thus, the present invention contemplates the detection of certain biomarkers followed by a change in the treatment of SLE, which may include using standard therapeutic approaches where indicated. In general, the treatment of SLE involves treating elevated disease activity and trying to minimize the organ damage that can be associated with increased inflammation and increased immune complex formation/deposition/complement activation. Foundational treatment can include corticosteroids and/or anti-malarial drugs. Certain types of lupus nephritis such as diffuse proliferative glomerulonephritis require bouts of cytotoxic drugs. These drugs include, most commonly, cyclophosphamide and mycophenolate. Hydroxychloroquine (HCQ) was approved by the FDA for lupus in 1955. Some drugs approved for other diseases are used for SLE "off-label". Belimumab (Benlysta) can be used as a treatment for elevated disease activity seen in autoantibody-positive lupus patients.

Due to the variety of symptoms and organ system involvement with SLE, its severity in an individual must be assessed in order to successfully treat SLE. Mild or remittent disease may, sometimes, be safely left minimally treated with hydroxychloroquine alone. If required, nonsteroidal anti-inflammatory drugs and low dose steroids may also be used. Hydroxychloroquine (HCQ) is an FDA-approved antimalarial used for constitutional, cutaneous, and articular manifestations. Hydroxychloroquine has relatively few side effects, and there is evidence that it improves survival among people who have SLE and stopping HCQ in stable SLE patients led to increased disease flares in Canadian lupus patients. Disease-modifying antirheumatic drugs (DMARDs) are often used off-label in SLE to decrease disease activity and lower the need for steroid use. DMARDs commonly in use are methotrexate and azathioprine. In more severe cases, medications that aggressively suppress the immune system (primarily high-dose corticosteroids and major immunosuppressants) are used to control the disease and prevent damage. Cyclophosphamide is used for severe glomerulonephritis, as well as other life-threatening or organ-damaging complications, such as vasculitis and lupus cerebritis. Mycophenolic acid is also used for treatment of lupus nephritis, but it is not FDA-approved for this indication.

Depending on the dosage, people who require steroids may develop Cushing's symptoms of truncal obesity, purple striae, buffalo hump and other associated symptoms. These may subside if and when the large initial dosage is reduced, but long-term use of even low doses can cause elevated blood pressure, glucose intolerance (including metabolic syndrome and/or diabetes), osteoporosis, insomnia, avascular necrosis and cataracts.

Numerous new immunosuppressive drugs are being actively tested for SLE. Rather than suppressing the immune system nonspecifically, as corticosteroids do, they target the responses of individual types of immune cells. Belimumab, or a humanized monoclonal antibody against B-lymphocyte stimulating factor (BlyS or BAFF), is FDA approved for lupus treatment and decreased SLE disease activity, especially in patients with baseline elevated disease activity and the presence of autoantibodies. Addition drugs, such as abatacept, epratuzimab, etanercept and others, are actively being studied in SLE patients and some of these drugs are already FDA-approved for treatment of rheumatoid arthritis or other disorders. Since a large percentage of people with SLE suffer from varying amounts of chronic pain, stronger prescription analgesics (pain killers) may be used if over-the-counter drugs (mainly nonsteroidal anti-inflammatory drugs) do not provide effective relief. Potent NSAIDs such as indomethacin and diclofenac are relatively contraindicated for patients with SLE because they increase the risk of kidney failure and heart failure.

Moderate pain is typically treated with mild prescription opiates such as dextropropoxyphene and co-codamol. Moderate to severe chronic pain is treated with stronger opioids, such as hydrocodone or longer-acting continuous-release opioids, such as oxycodone, MS Contin, or methadone. The fentanyl duragesic transdermal patch is also a widely used treatment option for the chronic pain caused by complications because of its long-acting timed release and ease of use. When opioids are used for prolonged periods, drug tolerance, chemical dependency, and addiction may occur. Opiate addiction is not typically a concern, since the condition is not likely to ever completely disappear. Thus, lifelong treatment with opioids is fairly common for chronic pain symptoms, accompanied by periodic titration that is typical of any long-term opioid regimen.

Intravenous immunoglobulins may be used to control SLE with organ involvement, or vasculitis. It is believed that they reduce antibody production or promote the clearance of immune complexes from the body, even though their mechanism of action is not well-understood. Unlike immunosuppressives and corticosteroids, IVIGs do not suppress the immune system, so there is less risk of serious infections with these drugs.

Avoiding sunlight is the primary change to the lifestyle of SLE sufferers, as sunlight is known to exacerbate the disease, as is the debilitating effect of intense fatigue. These two problems can lead to patients becoming housebound for long periods of time. Drugs unrelated to SLE should be prescribed only when known not to exacerbate the disease. Occupational exposure to silica, pesticides and mercury can also make the disease worsen.

Renal transplants are the treatment of choice for end-stage renal disease, which is one of the complications of lupus nephritis, but the recurrence of the full disease in the transplanted kidney is common in up to 30% of patients.

Antiphospholipid syndrome is also related to the onset of neural lupus symptoms in the brain. In this form of the disease the cause is very different from lupus: thromboses (blood clots or "sticky blood") form in blood vessels, which prove to be fatal if they move within the blood stream. If the thromboses migrate to the brain, they can potentially cause a stroke by blocking the blood supply to the brain. If this disorder is suspected in patients, brain scans are usually required for early detection. These scans can show localized areas of the brain where blood supply has not been adequate. The treatment plan for these patients requires anticoagulation. Often, low-dose aspirin is prescribed for this purpose, although for cases involving thrombosis anticoagulants such as warfarin are used.

Pharmaceutical Formulations and Delivery. A change in therapeutic application is contemplated, it will be necessary to prepare pharmaceutical compositions in a form appropriate for the intended application. Generally, this will entail preparing compositions that are essentially free of pyrogens, as well as other impurities that could be harmful to humans or animals.

Generally, appropriate salts and buffers are employed to render delivery vectors stable and allow for uptake by target cells. Buffers also will be employed when recombinant cells are introduced into a patient. Aqueous compositions described herein comprise an effective amount of the vector to cells, dissolved or dispersed in a pharmaceutically acceptable carrier or aqueous medium. Such compositions also are referred to as inocula.

As used herein, the phrases "pharmaceutically" or "pharmacologically acceptable", refer to molecular entities and compositions that do not produce adverse, allergic, or other untoward reactions when administered to an animal or a human. As used herein, "pharmaceutically acceptable carrier" includes any and all solvents, dispersion media, coatings, antibacterial and antifungal agents, isotonic and absorption delaying agents and the like. The use of such media and agents for pharmaceutically active substances is well known in the art. Except insofar as any conventional media or agent is incompatible with the vectors or cells described herein, its use in therapeutic compositions is contemplated.
Supplementary active ingredients also can be incorporated into the compositions.

The active compositions described herein may include classic pharmaceutical preparations. Administration of these compositions will be via any common route so long as the target tissue is available via that route. Such routes include oral, nasal, buccal, rectal, vaginal or topical route. Alternatively, administration may be by orthotopic, intradermal, subcutaneous, intramuscular, intraperitoneal, or intravenous injection. Such compositions would normally be administered as pharmaceutically acceptable compositions. The active compounds may also be administered parenterally or intraperitoneally. Solutions of the active compounds as free base or pharmacologically acceptable salts can be prepared in water suitably mixed with a surfactant, such as hydroxypropylcellulose. Dispersions can also be prepared in glycerol, liquid polyethylene glycols, and mixtures thereof and in oils. Under ordinary conditions of storage and use, these preparations contain a preservative to prevent the growth of microorganisms.

The pharmaceutical forms suitable for injectable use include sterile aqueous solutions or dispersions and sterile powders for the extemporaneous preparation of sterile injectable solutions or dispersions. In all cases the form must be sterile and must be fluid to the extent that easy syringability exists. It must be stable under the conditions of manufacture and storage and must be preserved against the contaminating action of microorganisms, such as bacteria and fungi. The carrier can be a solvent or dispersion medium containing, for example, water, ethanol, polyol (for example, glycerol, propylene glycol, and liquid polyethylene glycol, and the like), suitable mixtures thereof, and vegetable oils. The proper fluidity can be maintained, for example, by the use of a coating, such as lecithin, by the maintenance of the required particle size in the case of dispersion and by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminum monostearate and gelatin.

Sterile injectable solutions are prepared by incorporating the active compounds in the required amount in the appropriate solvent with various other ingredients enumerated above, as required, followed by filtered sterilization. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, the preferred methods of preparation are vacuum-drying and freeze-drying techniques which yield a powder of the active ingredient plus any additional desired ingredient from a previously sterile-filtered solution thereof.

As used herein, the phrase "pharmaceutically acceptable carrier" refers to any and all solvents, dispersion media, coatings, antibacterial and antifungal agents, isotonic and absorption delaying agents and the like. The use of such media and agents for pharmaceutical active substances is well known in the art. Except insofar as any conventional media or agent is incompatible with the active ingredient, its use in the therapeutic compositions is contemplated. Supplementary active ingredients can also be incorporated into the compositions.

For oral administration the polypeptides described herein may be incorporated with excipients and used in the form of non-ingestible mouthwashes and dentifrices. A mouthwash may be prepared incorporating the active ingredient in the required amount in an appropriate solvent, such as a sodium borate solution (Dobell's Solution). Alternatively, the active ingredient may be incorporated into an antiseptic wash containing sodium borate, glycerin and potassium bicarbonate. The active ingredient may also be dispersed in dentifrices, including: gels, pastes, powders and slurries. The active ingredient may be added in a therapeutically effective amount to a paste dentifrice that may include water, binders, abrasives, flavoring agents, foaming agents, and humectants.

Compositions for use as described herein may be formulated in a neutral or salt form. Pharmaceutically-acceptable salts include the acid addition salts (formed with the free amino groups of the protein) and which are formed with inorganic acids such as, for example, hydrochloric or phosphoric acids, or such organic acids as acetic, oxalic, tartaric, mandelic, and the like. Salts formed with the free carboxyl groups can also be derived from inorganic bases such as, for example, sodium, potassium, ammonium, calcium, or ferric hydroxides, and such organic bases as isopropylamine, trimethylamine, histidine, procaine and the like. Upon formulation, solutions will be administered in a manner compatible with the dosage formulation and in such amount as is therapeutically effective. The formulations are easily administered in a variety of dosage forms such as injectable solutions, drug release capsules and the like. For parenteral administration in an aqueous solution, for example, the solution should be suitably buffered if necessary and the liquid diluent first rendered isotonic with sufficient saline or glucose. In this connection, sterile aqueous media, which can be employed will be known to those of skill in the art in light of the present disclosure. For example, one dosage could be dissolved in 1 ml of isotonic NaCl solution and either added to 1,000 ml of hypodermoclysis fluid or injected at the proposed site of infusion, (see for example, "Remington's Pharmaceutical Sciences," 15th Ed., 1035-1038 and 1570-1580). Some variation in dosage will necessarily occur depending on the condition of the subject being treated. The person responsible for administration will, in any event, determine the appropriate dose for the individual subject. Moreover, for human administration, preparations should meet sterility, pyrogenicity, general safety, and purity standards as required by FDA Office of Biologies standards.

Kits are also described herein. Such kits can comprise a carrier, package or container that is compartmentalized to receive one or more containers such as vials, tubes, and the like, each of the container(s) comprising one of the separate elements to be used in the method, in particular, a Bright inhibitor. The kit will typically comprise the container described above and one or more other containers comprising materials desirable from a commercial end user standpoint, including buffers, diluents, filters, and package inserts with instructions for use. In addition, a label can be provided on the container to indicate that the composition is used for a specific therapeutic application, and can also indicate directions for either in vivo or in vitro use, such as those described above. Directions and or other information can also be included on an insert, which is included with the kit. In particular, kits described herein contemplate the assemblage of agents for assessing levels of the biomarkers discussed above along with one or more of an SLE therapeutic and/or a reagent for ANA testing and/or anti-ENA, as well as controls for assessing the same.

Current biomarkers in lupus disease activity measures have limited utility for reflecting heightened clinical disease activity. They are neither the earliest, nor most informative, measurements of clinical disease activity. Although SLE-associated autoantibody specificities such as anti-dsDNA, anti-spliceosome and anti-Ro/SSA, accumulate in SLE patients years before classification, their presence is not sufficient to reflect clinical disease activity and risk for permanent organ damage. ANAs are also found in sera from patients with other systemic rheumatic diseases, and from healthy individuals who do not go on to develop SLE, including some unaffected family members of SLE patients, and up to 14% of the general population. Because individuals may remain healthy despite being ANA-positive, ANA positivity alone is likely not the sole pathogenic driver of SLE. In addition to ANA positivity, the dysregulation of various immune pathways driven by soluble mediators may contribute to clinical disease activity.

EXAMPLES. The following examples are included to further illustrate various aspects of the invention. It should be appreciated by those of skill in the art that the techniques disclosed in the examples, which follow, represent techniques and/or compositions discovered by the inventor to function well in the practice of the invention, and thus can be considered to constitute preferred modes for its practice.

SLE is a complex autoimmune disease marked by immune dysregulation. A comprehensive but cost-effective tool to track relevant mediators of altered disease activity would help improve disease management and prevent organ damage. The goal of this example was to identify critical components of a practical biometric to distinguish active from low lupus disease activity.

The present invention includes the identification of certain immune mediators, including SLE-associated autoantibody specificities and inflammatory and regulatory soluble mediator pathways, potentially dysregulated with changes in SLE disease activity. Using a multiplex approach, SLE patients with low (hSLEDAI <4) or active (hSLEDAI≥4) disease activity visits, compared to matched healthy controls (HC) demonstrated disease activity-dependent changes in the accumulation of autoantibody specificities and plasma levels of inflammatory and regulatory mediators. These results enabled the development of a combined Lupus Disease Activity Immune Index (LDAII) that reflects immune alterations with changes in clinical disease activity.

SLE-linked immune mediators and autoantibodies were evaluated in 311 plasma samples from 198 patients with classified SLE procured on dates of low clinical disease (Systemic Lupus Erythematosus Disease Activity Index (SLEDAI) < 4, range 0-3, n=132) or more active disease (SLEDAI ≥ 4, range 4-30, n=179) as well as healthy controls (HC) matched for race, sex, and age (n=48). Thirty two soluble mediators and SLE-associated autoantibody specificities, including dsDNA, chromatin, Ro/SSA, La/SSB, Sm, SmRNP, and RNP, were assessed by multiplex bead-based assay or sandwich ELISA (BLyS, APRlL, and TGF-β). Soluble mediator levels were compared across clinical disease activity levels in conjunction with the presence of autoantibodies.

Patients with low or active disease were similar in age, ethnicity, and sex. After adjusting for multiple comparisons (Bonferroni corrected *p*<*0.0018*), IL-6, IL-1α, IP-10, and IL-8 were significantly correlated with SLEDAI scores (Spearman r=0.179-0.253), yet 22/32 soluble mediators significantly correlated with the number of SLE-associated autoantibodies accrued, including those listed above, as well as SCF, IFN-α, IFN-γ, IL-17A, IL-10, MIG, MIP-1β, TNFRII, and BLyS (r=0.318 [IL-17A]-0.468 [IP-10]). The regulatory mediator IL-10 was highest in samples (*p*<*0.05*) from patients with low disease activity, while inflammatory mediators were highest in active disease samples with accrued autoantibody specificities (*p*<*0.001*). The inventors integrated these findings to build a Lupus Disease Activity Immune Index (LDAII), calculated utilizing normalized (case vs. control) soluble mediator levels (n=32) weighted by the number of SLE-associated autoantibodies in each individual. The LDAII distinguished patients with clinically active (CA) vs. quiescent (CQ) disease who were either serologically (dsDNA binding and low complement) active (SA) or quiescent (SQ) (*p*<*0.0001*), whereby the number of accumulated autoantibodies (*p*<*0.0001*) as well as IL-6, IL-8, and IP-10 levels (*p*≤*0*.0006) were most significantly altered. In addition, the LDAII was able to differentiate clinically and serologically quiescent (CQSQ) SLE patients vs. HC (*p*=*0.019*). Finally, the LDAII significantly correlated with SLEDAI scores in patients (*p*<*0.0001*) and identified patients with renal organ involvement (*p*=*0.002*), in whom SCF, TNFRII, and MCP-1 were also most significantly altered (*p*≤*0.005*).

Study population, clinical data, and sample collection. Studies were performed in accordance with the Helsinki Declaration and approved by the Institutional Review Boards of the Oklahoma Medical Research Foundation and the University of Oklahoma Health Sciences Center. Undiluted plasma and serum samples were procured from 198, appropriately consented, SLE patients (meeting > 4 ACR classification criteria (39)) in the Oklahoma Lupus Cohort and 48 race and sex-matched healthy individuals (controls or Ctls) in the Oklahoma Immune Cohort, Table 1. Samples were stored at -80° in the Oklahoma Rheumatic Diseases Research Core Center (ORDRCC), CAP-certified, biorepository at OMRF. Samples in the biorepository have been tested with respect to shipment time and method, processing procedures, storage conditions, and length of storage for the ability to determine levels of soluble mediators and SLE-associated AutoAbs in samples from SLE patients and Ctls (33, 36, 37, 40-45).

**Table 1. Study Participant Demographics**

| | | **SLE^{a}** | **Healthy Control^{b}** | **p-value^{b}** |
|---|---|---|---|---|
| **# of individuals** | | 198 | 48 | -- |
| **Gender** | | | | *0.2652* |
| | **Female (%)** | 178 (90%) | 46 (96%) | -- |
| **Race (n, %)** | | | | *0.4234* |
| | **EA** | 112 (57%) | 24 (50%) | -- |
| | **non-EA^{d}** | 86 (43%) | 24 (50%) | -- |
| **# of samples** | | 311 | 48 | -- |
| **Age at Draw (SD)** | | 43 (12) | 44 (15) | *0.5605* |

| | | | | |
|---|---|---|---|---|
| ^{a}SLE patients met ≥ 4 classification criteria for SLE (ACR and SLICC) ^{b}Matched to SLE by Race, Gender, and Age ^{c}Statistical significance (*p*≤*0.05*) determined by paired t-test (continuous data) or Fisher's exact test (categorical data) ^{d}non-EA = African-American, Asian, American Indian, or multi-racial | | | | |

Demographic and clinical information were collected as previously described (39), including medication usage, clinical laboratory values, and clinical disease activity. The presence of organ system involvement was evaluated by the administration of the hybrid Systemic Lupus Erythematosus Disease Activity Index (hSLEDAI; SELENA-SLEDAI with proteinuria as defined by SLEDAI-2K) (32, 33), including the presence of disease manifestations involving the central nervous system (CNS; seizure, psychosis, organic brain syndrome, visual disturbance, cranial nerve disorder, or lupus headache), vasculitis, arthritis, myositis, nephritis (urinary casts, hematuria, proteinuria, or pyuria), mucocutaneous damage (rash, alopecia, or mucosal ulcers), serositis (pleuritis or pericarditis), or hematologic manifestations (low complement, increased DNA binding, fever, thrombocytopenia, or leukopenia) (16). Patient visits with hSLEDAI scores < 4 were as "low" disease activity, while patient visits with hSLEDAI scores ≥ 4 were considered as "active" disease. Blood samples were procured from each participant at each clinical visit. Plasma and serum samples were stored at -20°C until time of assay. Assays were performed on freshly thawed samples.

Measurement of soluble mediators and autoantibody specificities. Plasma soluble mediators (n=32, Table 2) were examined, including cytokines, chemokines, and soluble receptors, using validated multiplex bead-based assay or ELISAs (BLyS, APRIL, and TGF-β) (46). Data were analyzed on the Bio-Rad BioPlex 200^{®} array system (Bio-Rad Technologies, Hercules, CA), with a lower boundary of 100 beads per sample/analyte. Median fluorescence intensity for each analyte was interpolated from 5-parameter logistic nonlinear regression standard curves. Analytes below the detection limit were assigned a value of 0.001 pg/mL. A known control serum was included on each plate (Cellgro human AB serum, Cat#2931949, L/N#M1016). Mean inter-assay coefficient of variance (CV) of multiplexed bead-based assays for cytokine detection has previously been shown to be 10-14% (47, 48), and a similar average CV (10.5%) across the analytes in this assay was obtained using healthy control serum. Intra-assay precision of duplicate wells averaged <10% CV in each xMAP assay.

**Table 2. Plasma Mediators Assessed in SLE Patient and Control Samples**

| **Innate** | **Thl-like** | **TNFR Superfamily** |
|---|---|---|
| **IL-1a** | **IL-12 (p70)** | **BLyS*** |
| **IL-1b** | **IFN-g** | **APRIL*** |
| **IL-1RA** | **IL-2** | **sCD40L** |
| **IFN-a** | **IL-2Ra** | **TNFRI (p55)** |
| | | **TNFRII (p75)** |
| **Other** | **Th-17 like** | **TRAIL** |
| **SCF** | **IL-17A** | **TWEAK** |
| | **IL-21** | |
| | **IL-6** | **Chemokine/Adhesion molecules** |
| | | **IL-8/CXCL8** |
| **Regulatory** | **Th2-like** | **IP-10/CXCL10** |
| **IL-10** | **IL-5** | **MIP-1a/CCL3** |
| **TGF-b*** | **IL-13** | **MIP-1b/CCL4** |
| **LAP (TGF-b)** | | **MCP-1/CCL2** |
| | | **MCP-3/CCL7** |
| | | **MIG/CXCL9** |
| ***assessed by ELISA** | | **ICAM-1** |

Serum samples were screened for autoantibody specificities using the BioPlex 2200 multiplex system (Bio-Rad Technologies, Hercules, CA). The BioPlex 2200 ANA kit uses fluorescently dyed magnetic beads for simultaneous detection of 11 autoantibody specificity levels, including reactivity to dsDNA, chromatin, ribosomal P, Ro/SSA, La/SSB, Sm, the Sm/RNP complex, RNP, Scl-70, centromere B, and Jo-1 (49). SLE-associated autoantibody specificities to dsDNA, chromatin, Ro/SSA, La/SSB, Sm, Sm/RNP complex, and RNP were used for analysis in the current study. Anti-dsDNA (IU/mL) has a previously determined positive cutoff of 10 IU/mL; an Antibody Index (AI) value (range 0-8) is reported by the manufacturer to reflect the fluorescence intensity of each of the other autoantibody specificities with a positive cutoff of AI=1.0. The AI scale is standardized relative to calibrators and control samples provided by the manufacturer.

Statistical Analyses. Categorical variables were compared by Fisher's exact test. Disease activity scores in low vs. active disease clinical visits were compared using unpaired t-test. Number of autoantibody specificities and plasma soluble mediator concentrations were compared between SLE patient visits with low or active disease by Mann-Whitney test. Plasma mediator concentrations and number of autoantibody specificities were compared between SLE patient visits with low and active disease and race/sex matched Ctl samples by Kruskal-Wallis test with Dunn's multiple comparison. Plasma mediator concentrations were correlated with hSLEDAI scores or number of autoantibody specificities by Spearman's rank correlation. Except where noted, analyses were performed using GraphPad Prism 6.02 (GraphPad Software, San Diego, CA).

A Lupus Disease Activity Immune Index (LDAII) calculation was developed to compare the overall level of inflammation during patient visits with varying levels of disease activity, distinguished from Ctls. The LDAII calculation followed an approach that the inventors have similarly used to develop a Lupus Flare Prediction Index (LFPI) used for SLE patients (42, 45). The LDAII summarizes the dysregulation of all 32 plasma mediators assessed in SLE patients at clinic visits with low and active disease and matched Ctls, weighted by their correlation to the number of AutoAb specificities detected from samples procured at the same visit. The LDAII was calculated as follows: 1. The concentrations of all 32 baseline plasma mediators, Table 2, were log-transformed for each SLE patient or Ctl visit. 2. Each log-transformed soluble mediator level for each participant visit was standardized: (observed value)-(mean value of all SLE patient and Ctl visits)/(standard deviation of all SLE patient and Ctl visits) 3. Spearman coefficients were generated from a linear regression model testing associations between the number of AutoAb specificities each soluble mediator assessed in all SLE patient and Ctl visits (Spearman r); 4. The transformed and standardized soluble mediator levels at were weighted (multiplied) by their respective Spearman coefficients (Spearman r). 5. For each participant visit, the log transformed, standardized and weighted values for each of the 32 soluble mediators were summed to calculate a total LDAII.

The LDAII was compared between SLE patient visits with low and active disease by Mann-Whitney test, and additionally to Ctls by Kruskal-Wallis test with Dunn's multiple comparison. Odds ratio (OR) was determined for the likelihood of SLE patient visits with active disease vs. low disease activity (or Ctl) to have a positive or negative LDAII score, respectively; significance for was determined by Fisher's exact test.

Increased number of AutoAb specificities and altered levels of select mediators with clinically active disease.

The inventors have previously demonstrated that patients who transition to classified SLE (meeting > 4 ACR classification criteria (39)) exhibit multiple levels of immune dysregulation, including accumulation of SLE-associated AutoAb specificities (36, 37, 50) and alteration of both inflammatory and regulatory soluble mediators (36, 37). Based on this hypothesis that such immune dysregulation would also be reflected in SLE patients with clinically active disease, the inventors compared serum autoantibody and plasma soluble mediator profiles in samples procured during clinic visits whereby 198 SLE patients exhibited low disease activity (hSLEDAI < 4, range 0-3, n=132) or active disease (hSLEDAI ≥ 4, range 4-30, n=179), Figs. 1A and 1B and Table 3. As expected, SLE patients with active disease had significantly higher hSLEDAI scores (Fig. 1A) and were more likely to exhibit various organ system manifestations (Table 3) than SLE patients with low disease activity. In addition, SLE patients with active disease exhibited a significant increase in the number of SLE-associated AutoAb specificities compared to the number detected during low disease activity (p=0.0038, Fig. 1B), while SLE patients had significantly more AutoAb specificities compared to matched Ctls (p<0.0001), irrespective of disease activity. AutoAbs against dsDNA (p<0.0001), chromatin (p=0.0005), and Sm (p=0.0380) were more likely to be positive during periods of active disease, while the frequency of Ro/SSA, La/SSB, SmRNP, and RNP AutoAb specificities were similar in low and active disease, Table 3. SLE patients with active disease were more likely to be prescribed steroids (p<0.0001) and similarly prescribed hydroxychloroquine and/or immunosuppressants as patients with low disease activity, Table 3.

**Table 3. SLE Patient Medication, Autoantibody, and Clinical Characteristics**

| **Disease Activity^{a}:** | **Low** | **Active** | **p-value^{b}** |
|---|---|---|---|
| **# of visits/samples** | 132 | 179 | -- |
| **Age at Draw (SD)** | 45 (13) | 41 (12) | 0.2814 |
| | | | |

| **Medications at Draw, # (%)** | | | |
|---|---|---|---|
| **Steroids** | 41 (31%) | 103 (57%) | **<*0.0001*** |
| **Hydroxychloroquine** | 93 (70%) | 119 (66%) | *0.5382* |
| **Immunosuppressants^{c}** | 43 (33%) | 53 (30%) | *0.6200* |
| **Major Immunosuppressants^{d}** | 18 (14%) | 34 (19%) | *0.2230* |
| | | | |

| **AutoAb Specificities, # (%)^{e}** | | | |
|---|---|---|---|
| **dsDNA** | 12 (9%) | 57 (32%) | ***<0.0001*** |
| **Chromatin** | 27 (20% | 71 (40%) | ***0.0005*** |
| **Ro/SSA** | 44 (33%) | 68 (38%) | *0.5474* |
| **La/SSB** | 17 (13%) | 26 (15%) | *0.8678* |
| **Sm** | 17 (13%) | 41 (23%) | ***0.0380*** |
| **SmRNP** | 28 (21%) | 52 (29%) | *0.1493* |
| **RNP** | 32 (24%) | 56 (31%) | *0.2506* |
| | | | |

| **hSLEDAI organ system manifestations: n positive (%)** | | | |
|---|---|---|---|
| **CNS^{f}** | 0 | 0 | -- |
| **Arthritis** | 0 | 98 (55%) | **<*0.0001*** |
| **Renal^{g}** | 0 | 24 (13%) | **<*0.0001*** |
| **Mucocutaneous^{h}** | 24 (18%) | 128 (72%) | **<*0.0001*** |
| **Serositisⁱ** | 1 (1%) | 9 (5%) | ***0.0483*** |
| **Serologic^{j}** | 32 (24%) | 112 (63%) | **<*0.0001*** |
| **Hematalogic^{k}** | 4 (3%) | 13 (7%) | *0.1322* |

| | | | |
|---|---|---|---|
| ^{a}Low = hSLEDAI < 4; Active = hSLEDAI ≥ 4 ^{b}Statistical significance (***p*≤*0.05***) determined by paired t-test (continuous data) or Fisher's exact test (categorical data) ^{c}Immunosuppressants = methotrexate, azathioprine ^{d}Major immunosuppressants = mycophenolate mofetil, cyclophosphamide ^{e}determined by Bioplex^{®} 2200 ANA Screen ^{f}CNS = seizure, psychosis, organic brain syndrome, visual disturbance, cranial nerve disorder, lupus headache, CVA ^{g}Renal = urinary casts, hematuria, proteinuria, pyuria ^{h}Mucocutaneous = rash, alopecia, mucosal ulcers ⁱSerositis = pleurisy, pericarditis ^{j}Serologic = low complement, increased DNA binding ^{k}Hematologic = thrombocytopenia, leukopenia | | | |

In addition to changes in AutoAb specificities, the inventors also evaluated whether SLE patients with active disease also had alterations in SLE-associated soluble mediators, Table 4 and Figs. 2A to 2I. After adjusting for multiple comparison, plasma levels of select mediators, including IL-8, IP-10, IL-1α, and IL-6 directly correlated with hSLEDAI scores at clinic visits concurrent with sample procurement, Table 4. This was further reflected by increased levels of plasma mediators in SLE patients with active disease. The type I IFN, IFN-α (Fig. 2A), along with the IFN-driven chemokine, IP-10 (Fig. 2B), and the innate mediator, IL-6 (Fig. 2C), were all significantly higher in SLE patient samples from periods of active disease compared to low disease activity, and SLE patients had higher levels of these mediators than Ctls, irrespective of clinical disease activity (p< 0.05). The innate mediator, IL-1α (Fig. 2D), chemokine IL-8 (Fig. 2E), and Th17-type mediator, IL-21 (Fig. 2F), were highest in SLE patients with active disease (p<0.05), while patients with low disease activity had similar levels as Ctls. SLE patients exhibited higher levels of BLyS than Ctls (p<0.001), irrespective of disease activity, which was highest, albeit insignificant, in SLE patients with active disease, Fig. 2G. The regulatory mediator, IL-10 (Fig. 2H), was highest in SLE patients with low disease activity (SLE patients had significantly higher levels than Ctls, p<0.01), while TGF-β levels where highest in Ctls (p<0.01) and similar between SLE patients with low and active disease, Fig. 2I. These data indicate that a number of select mediators may be altered in direct association with clinically active SLE.

**Table 4. Distinct Plasma Mediators Correlate with Disease Activity**

| **Mediator** | **Spearman r** | **95% CI** | ***p value^{a}*** |
|---|---|---|---|
| **IL-8** | 0.253 | 0.143 to 0.357 | ***<0.0001*** |
| **IP-10** | 0.181 | 0.068 to 0.290 | ***0.0013*** |
| **IL-la** | 0.180 | 0.067 to 0.290 | ***0.0014*** |
| **IL-6** | 0.179 | 0.066 to 0.287 | ***0.0016*** |
| **IL-21** | 0.156 | 0.042 to 0.266 | *0.0058* |
| **IFN-a** | 0.132 | 0.015 to 0.240 | *0.0191* |
| **IL-12(p70)** | 0.131 | 0.017 to 0.242 | *0.0209* |
| **MIG** | 0.129 | 0.015 to 0.240 | *0.0228* |
| **MIP-la** | 0.126 | 0.011 to 0.237 | *0.0267* |
| **MIP-lb** | 0.122 | 0.008 to 0.234 | *0.0311* |
| **IL-2Ra** | 0.122 | 0.008 to 0.233 | *0.0313* |
| **TRAIL** | 0.121 | 0.007 to 0.233 | *0.0324* |
| **TNFRII** | 0.119 | 0.004 to 0.230 | *0.0361* |

| | | | |
|---|---|---|---|
| ^{a}Spearman correlation, Bonferroni corrected significance ***p*=*0.0038*** | | | |

Soluble mediator levels are altered in association with the presence of AutoAbs and clinical disease activity. Given the increased number of AutoAb specificities and altered levels of select plasma mediators detected in SLE patients with active disease, the inventors queried whether soluble mediator levels correlated with the number of AutoAb specificities detected, Table 5. The inventors found that multiple additional soluble mediators (22/32 assessed) significantly (p≤0.0010) correlated with the number of accumulated SLE-associated AutoAbs present in SLE patients and matched healthy controls, including innate and adaptive mediators, chemokines, and TNF superfamily members. This led us to evaluate how levels of these soluble mediators compare between SLE patients with either low or active disease who are also either AutoAb negative (Neg) or positive (Pos) vs. Ctls (Figs. 3-6). Both type I (IFN-α, Fig. 3A) and type II (IFN-γ, Fig. 3B) IFNs are increased in AutoAb Pos SLE patients, with a significant difference between AutoAb Neg and Pos patients with active disease; AutoAb positive SLE patients with active disease had the highest plasma levels of these mediators compared to Ctls (p<0.001). For the IFN chemokines IP-10 (Fig. 3C) and MIG (Fig. 3D), there was a significant increase in these mediators between AutoAb Pos and Neg SLE patients with either low (p<0.05) or active (p<0.0001) disease and the highest levels in AutoAb Pos SLE patients with active disease compared to Ctls (p<0.001). These data suggest that in addition to possibly being directly associated with altered disease activity (Fig. 2), IFN-associated mediators may also be tied to the accumulation of AutoAb specificities (Figs. 3A to 3D).

**Table 5. Plasma Mediators Correlate with Number of SLE-Associated Autoantibody Specificities**

| **Mediator** | **Spearman r** | **95% CI** | ***p value^{a}*** | | **Mediator** | **Spearman r** | **95% CI** | ***p value^{a}*** |
|---|---|---|---|---|---|---|---|---|
| **IP-10** | 0.468 | 0.301 to 0.547 | **<*0.0001*** | | **IL-12(p70)** | 0.242 | 0.139 to 0.339 | **<*0.0001*** |
| **TNFRII** | 0.388 | 0.294 to 0.475 | **<*0.0001*** | | **MIP-1α** | 0.233 | 0.130 to 0.332 | **<*0.0001*** |
| **BLyS** | 0.386 | 0.291 to 0.473 | **<*0.0001*** | | **IFN-γ** | 0.226 | 0.123 to 0.325 | **<*0.0001*** |
| **IL-2Ra** | 0.354 | 0.257 to 0.444 | **<*0.0001*** | | **SCF** | 0.225 | 0.122 to 0.324 | **<*0.0001*** |
| **MIP-1β** | 0.338 | 0.240 to 0.429 | **<*0.0001*** | | **IFN-α** | 0.221 | 0.117 to 0.320 | **<*0.0001*** |
| **IL-8** | 0.330 | 0.232 to 0.422 | **<*0.0001*** | | **IL-1RA** | 0.789 | 0.073 to 0.280 | **<*0.0001*** |
| **TRAIL** | 0.319 | 0.220 to 0.411 | **<*0.0001*** | | **MCP-3** | 0.174 | 0.069 to 0.276 | ***0.0009*** |
| **IL-1α** | 0.303 | 0.203 to 0.397 | **<*0.0001*** | | **IL-17A** | 0.174 | 0.068 to 0.275 | ***0.0010*** |
| **IL-5** | 0.296 | 0.195 to 0.390 | **<*0.0001*** | | **Total TGF-β** | 0.157 | 0.051 to 0.259 | *0.0029* |
| **MIG** | 0.295 | 0.194 to 0.390 | **<*0.0001*** | | **TNFRII** | 0.149 | 0.043 to 0.252 | *0.0046* |
| **APRIL** | 0.292 | 0.191 to 0.386 | **<*0.0001*** | | **IL-21** | 0.143 | 0.037 to 0.246 | *0.0067* |
| **IL-6** | 0.289 | 0.188 to 0.384 | **<*0.0001*** | | **IL-2** | 0.136 | 0.030 to 0.239 | *0.0099* |
| **IL-10** | 0.248 | 0.145 to 0.345 | **<*0.0001*** | | **IL-1β** | 0.118 | 0.012 to 0.222 | *0.0251* |
| **MCP-1** | 0.246 | 0.143 to 0.344 | **<*0.0001*** | | **IL-13** | 0.115 | 0.009 to 0.219 | *0.0293* |

| | | | | | | | | |
|---|---|---|---|---|---|---|---|---|
| ^{a}Spearman correlation, Bonferroni corrected significance ***p*=*0.0018*** | | | | | | | | |

B-lymphocyte stimulator (BLyS) is a TNF superfamily member that is secreted in response to type I (51) and type II (52) IFNs and supports B cell survival and differentiation, contributing to autoantibody production and class switching (53). Similar to IFN-associated mediators, BLyS (Fig. 4A) is increased in AutoAb Pos SLE patients with either low or active disease (p<0.01), whereby AutoAb Pos SLE patients have the highest levels compared to Clts (p<0.0001). Another TNF superfamily member that is related to BLyS with respect to B-lymphocyte activation and AutoAb production, a proliferation-inducing ligand (APRIL, Fig. 4B)(53), is similarly increased in AutoAb Pos SLE patients with active disease (p<0.0001). Interestingly, comparing SLE patients with low vs. active disease, APRIL levels were found to be lower in AutoAb Neg SLE patients with active disease vs. their AutoAb positive counterparts, where APRIL was found to highest in SLE patients with active disease (p<0.05). Other TNF superfamily members assessed, including TRAIL (Fig. 4C) and TNFRII (Fig. 4D), were also increased in AutoAb Pos SLE patients with active disease (p<0.0001). Given that these two mediators also correlated to some extent with disease activity (Table 4), it is possible that TNF superfamily members may contribute to both AutoAb production and heightened clinical disease activity.

Other innate and adaptive mediators have been shown in the inventors' previous studies to be altered in SLE pathogenesis and heightened clinical disease (36, 42, 45). The inventors observed that innate mediators, including IL-1a (Fig. 5A, p<0.01) and IL-6 (Fig. 5B, p<0.05) are increased AutoAb Pos SLE patients with either low or active disease. In addition, IL-la distinguishes low vs active disease in AutoAb Pos SLE patients (Fig. 5A, p=0.0206). Increased levels of adaptive mediators, including the Th1-type mediator, IL-2 (Fig. 5C), and the Th17-type mediator, IL-21 (Fig. 5D), were also significantly elevated in AutoAb Pos SLE patients with active disease (p<0.05). Other inflammatory mediators were also elevated, including SCF (Fig. 6A, p=0.0356) and IL-8 (Fig. 6B, p<0.0001). Although plasma levels of IL-10 (Fig. 6C) were highest in AutoAb Neg SLE patients with low disease activity, possibly reflective of its regulatory attributes, there was a significant difference in IL-10 levels between AutoAb Neg and Pos SLE patients with active disease (p=0.0089), with higher levels in AutoAb Pos SLE patients, suggesting that IL-10 may also be acting in its capacity as a pro-inflammatory, positive regulator of B-lymphocyte activation and AutoAb production (54, 55). Similarly, although TGF-β (Fig. 6D) is highest in Ctls, reflecting inhibited regulation in SLE patients (36, 42, 45), there are higher levels in AutoAb Pos vs. Neg SLE patients with active disease (p=0.0066). This may be reflective of the ability of TGF-β, when secreted in conjunction with IL-6 (Fig. 5B), to drive pro-inflammatory, Th17-type responses (56, 57).

A weighted Lupus Disease Activity Immune Index (LDAII) differentiates active disease in SLE patients. Given the significant alterations in multiple immune pathways associated with active clinical disease in SLE patients, the inventors developed a Lupus Disease Activity Immune Index (LDAII) to summarize the immune dysregulation in individual patients, comparing low and active disease in SLE patients vs. the immune profile found in healthy individuals or controls (Ctls), Figs. 7A and 7B. The LDAII is calculated using log-transformed, normalized levels of each soluble mediator weighted based on their correlations to the number of SLE-associated AutoAb specificities detected. The sum of the weighted, log-transformed, normalized levels for each analyte produces the global LDAII (see Materials and Methods for details). A distinct advantage of this approach is that it does not require cut-offs for each cytokine/chemokine to establish positivity, and gives impact to those untransformed mediators with stronger (Spearman) correlations to accumulation of AutoAb specificities that would be found in SLE patients with active disease. The LDAII (calculated as a total of sub-scores contributed by each soluble mediator) was significantly higher in SLE patients with active clinical disease compared to SLE patients with low disease activity and Ctl samples (Fig. 7A). Including healthy individuals in the algorithm allows for optimal differentiation of SLE patients with low disease activity. Importantly, the LDAII significantly correlated with hSLEDAI scores (r=0.226, p<0.0001), differentiated SLE patients with active disease or low disease activity vs. Ctl (AUC = 0.671, p=0.0001), and carried a positive predictive value (PPV) of 0.94, with 79% specificity and 49% sensitivity, Fig. 7B. These data suggest that additional immune dysregulation is associated with clinical disease activity, and this heterogeneous dysregulation can be harnessed to form an LDAII with the potential to impact clinical care.

The LDAII differentiates SLE patients with Clinically and Serologically Active and Quiescent disease. In addition to assessing differences in immune dysregulation based on clinical disease activity (hSLEDAI) scores, the inventors also evaluated whether or not the LDAII could differentiate clinically active (CA) vs. quiescent (CQ) disease in combination with serologically active (SA) vs. quiescent disease (SQ), which is defined as meeting Immunologic disease activity criteria (anti-dsDNA binding AutoAbs and/or low complement levels) (14, 15) vs. Ctls (FIGS. 8A to 8F). Those SLE patients who had clinically active disease (CASA) had significantly higher LDAII scores than patients with clinically quiescent disease (CQSQ). Both SLE patient groups were significantly differentiated from Ctls (Fig. 8A), with a PPV of 0.88, 79% specificity, and 71% sensitivity (AUC = 0.804 [p<0.0001]), Fig. 8B. These data suggest that an immune index informed by a number of heterogeneously altered immune pathways in SLE patients can identify patients with heightened clinical disease activity. In addition, the inventors observed increased levels of mediators in CASA SLE patients (p<0.01), including IL-6 (Fig. 8C), IL-8 (Fig. 8D), and IP-10 (Fig. 8E), as well as an increased number of SLE-associated AutoAb specificities (Fig. 8F). Of interest, many CQSQ patients are still positive for at least one AutoAb specificity and have inflammatory mediators that are higher than Ctls, despite being both clinically and serologically quiescent.

The LDAII differentiates SLE patients with renal organ manifestations of disease activity. Chronic immune dysregulation, including pathogenic autoantibody production and increased levels of inflammatory mediators, contributes to progressive end-organ damage. Kidney damage and lupus nephritis are among most severe sequelae of SLE, contributing substantially to SLE-related morbidity and mortality (58). Early detection and treatment of kidney damage are imperative to minimize the risk of permanent organ damage and to preserve renal function. The inventors assessed whether the LDAII could differentiate SLE patients with renal involvement (FIGS. 9A to 9E), which was present in SLE patients with active disease (Table 3). The inventors found that SLE patients with active disease and renal manifestations had significantly higher LDAII scores than SLE patients with active disease without renal manifestations (p=0.0099), Fig. 9A. SLE patients with active disease, either with (p=0.0002) or without (p=0.0359) renal manifestation had higher LDAII scores than SLE patients with low disease activity(Fig. 9A), with a NPV of 0.96, 49% specificity, and 88% sensitivity (AUC = 0.689 [p=0.0029]), Fig. 9B. Both SCF (Fig. 9C) and TNFRII (Fig. 9D), as well as the number of AutoAb specificities (Fig. 9E) were increased is SLE patients with active disease and renal manifestation compared to those patients not exhibiting renal manifestations of disease activity (p<0.001 vs. Non-renal/active disease; p<0.01 vs. low disease activity visits). In addition, SLE patients with active disease, but no renal manifestations had more SLE-associated AutoAb specificities present than SLE patients with low disease activity (p=0.0261, Fig. 9E). These data suggest that harnessing immune dysregulation to inform the LDAII may be useful in identifying SLE patients with renal manifestations who would likely benefit from closer clinical monitoring to prevent the development, morbidity, and early mortality from lupus nephritis and end stage renal disease (ESRD).

Persistently active disease is burdensome for SLE patients and affects quality of life (59), even for those patients with longstanding disease (19). Disease activity has been shown to increase over time and the majority of patients with low disease activity will develop active disease (19) that is associated with both organ damage and early mortality (24, 25, 60) and highlights gaps in the optimal management of SLE. The ultimate goal is to move patients toward low disease activity where it has been shown to improve outcomes and prognosis (61), with fewer organ manifestations and less permanent organ damage, fewer treatments that carry significant side effects and morbidity, and lower mortality.

Recognition and early treatment to prevent tissue and organ damage is challenging, as signs and symptoms of disease activity are captured after their occurrence. In addition, long-term use of steroids (23) and other immune-suppressants (27) required to manage disease activity are associated with increased morbidity. The inability to proactively manage clinical disease limits medical care to reactive treatment, precluding proactive strategies of adding or increasing steroid-sparing immune modifying agents (28) to prevent end-organ damage (6-8) and reduce the pathogenic and socioeconomic burdens of SLE (29). The inventors have previously demonstrated that dysregulation of multiple immune pathways (36, 37), alongside the accumulation of SLE-associated AutoAb specificities (36, 37, 50), underlies the development and transition to classified SLE. The inventors have further shown that additional immune dysregulation occurs prior to clinical disease flare and the heterogeneity of immune pathway dysregulation and clinical disease could be harnessed by the advent of a lupus flare prediction index (42, 45). Of interest, this index did not inform concurrent clinical disease activity (42, 45).

The inventors sought to determine underlying immune dysregulation that reflects ongoing clinical disease activity and harness the information to inform an immune index that complements current clinical disease activity instruments. Within the current study, the inventors observed heterogeneity in the number and type of immune pathways altered in SLE patient samples from active vs. low disease activity. This may explain, in part, the variability among previous reports of inflammatory mediators in SLE patients with active disease (62-64), as well as the inconsistent correlations with and limited clinical utility of proposed serologic markers of disease activity, alone or in combination, including anti-dsDNA, complement, complement split products, and inflammatory markers (ESR and CRP) (65-67). Despite the heterogeneity in immune pathway involvement, each patient demonstrated elevations in inflammatory mediators from at least one pathway. It was not surprising that IFN-associated mediators were affected, including alterations in type I IFN (IFN-α), type II IFN (IFN-γ), and IFN-associated chemokines (IP-10, given the well described IFN signature present in SLE patients (68). IFN-associated mediators were affected directly in relation to disease activity (low vs. active, clinically/serologically quiescent vs. active), as well as in relation to the presence of SLE-associated AutoAbs, in a subset of patients. This finding is supported by previous studies that indicated that the IFN pathway can reflect disease activity (69), is dependent on the presence of AutoAbs in a subset of patients (70), but does not universally explain concurrent clinical disease activity (71).

There were a number of mediators that did not directly correlate with disease activity measures, yet correlated with the accumulation of AutoAb specificities and were able to differentiate low vs. active disease clinic visits within AutoAb positive vs. negative SLE patients. Notable in this category were the TNF superfamily mediators, BLyS and APRIL, that contribute to B-lymphocyte activation and AutoAb production (72-74). BLyS levels can arise from early dysregulation of type I (51) and type II (52) IFN mediators interacting with the accumulation of lupus-associated autoantibody specificities (37). The inventors observed that BLyS, as well as APRIL, differentiated AutoAb negative vs. positive SLE patients with active disease similarly to IFN-associated mediators (Figures 3-4). Yet, APRIL was also able to differentiate disease activity (low vs active) in AutoAb negative patients (active < low disease activity) and AutoAb positive patients (active > low disease activity). This divergence from BLyS in some instances (AutoAb negative samples) and convergent in others (AutoAb positive samples) is supported by other studies in SLE (72, 75-78) and may be explained by the unique and overlapping receptors that BLyS and APRIL utilize (72, 73, 79-82). Those receptors/pathways that BLyS and APRIL share likely lead to convergent downstream events (AutoAb production), while divergent receptor-signaling pathway mechanisms (e.g. APRIL-TACI interactions (80)) can contribute to APRIL's apparent role in negative regulation, as the inventors saw in AutoAb negative SLE samples (Figs. 4A to 4D).

Another pair of soluble mediators with ostensibly dual anti- and pro-inflammatory functions with respect to lupus disease activity are IL-10 and TGF-β. Both of these mediators have been shown to have regulatory functions (83-88) and the inventors saw that IL-10 is highest in AutoAb negative SLE patients with low disease activity (Figs. 6A to 6D), while TGF-β is highest in healthy controls, indicating a loss of regulation in SLE patients (87, 88). Conversely, SLE patients with active disease have increased levels of IL-10 and TGF-β in AutoAb positive (vs. negative) samples (Figs. 6A to 6D). IL-10 has been shown to have pro-inflammatory properties with respect to B-lymphocyte activation and AutoAb production (89), while TGF-β has been shown to contribute to Th17-type responses in the presence of IL-6 (90, 91) that leads to IL-21 secretion and stimulation of B-lymphocytes that contributes to AutoAb production and SLE pathogenesis (92, 93).

These immune system changes, in conjunction with lessons learned forming a lupus flare prediction index (42, 45), have guided us to develop a Lupus Disease Activity Immune Index (LDAII) that is informed by soluble mediator alterations and weighted by their correlation with SLE-associated AutoAb accumulation. Time and need for specialized training to minimize variability in disease activity assessment (31, 35) often limit the use of validated SLE disease activity measures in routine clinical practice. The detection of immune system changes associated with ongoing clinical disease activity, harnessed to inform the LDAII, would allow for the identification of patients in need of closer monitoring and enable early intervention with immune modifying treatments to prevent end-organ damage (6-8). A positive score would indicate a need for more frequent monitoring (94) and/or a change in medication to tamper ongoing inflammation prior to the onset of new or worsening clinical manifestations. A negative score would indicate maintenance of ongoing monitoring and medication schedules (they are working), the need for less frequent monitoring (particularly if visits occur at least quarterly), and/or consideration of tapering steroids (23) or other immune modifying agents (27) that carry significant side effects. It has been shown that SLE patients who participate more actively in their clinical care have less permanent organ damage (95). The LDAII allows patients to be monitored for their immune activity that precludes clinical disease activity and alerts them and their health care provider to the need for further clinical assessment.

Clinically quiescent SLE patients at risk for heightened disease activity and must be regularly monitored (14). Although SLE patients with active disease were more likely to meet hSLEDAI serologic criteria (increased DNA binding and/or hypocomplementemia) and be positive for anti-dsDNA AutoAbs (Table 3), neither of these factors have been shown to be predictors of heightened clinical disease activity (14, 42, 45). Importantly, the LDAII was able to differentiate patients with clinically and serologically quiescent (CQSQ) vs. active (CASA) disease (Figs. 8A to 8F), with a greater difference in IL-6, IL-8, and IP-10 levels, as well as number of SLE-associated AutoAbs present between CQSQ and CASA SLE patients than using hSLEDAI disease activity scores alone (Figs. 1-2). In addition, the LDAII was able to differentiate SLE patients with active disease who exhibited renal manifestations (Figs. 9A to 9E). The present invention allows the analysis of pathway-specific immune dysregulation to enable personalized, precision medicine for SLE patients with renal manifestations and lupus nephritis (LN). Further, the present invention allows for identifying individuals at greatest risk of developing LN and for defining measures of pathway-specific immune dysregulation to select the most appropriate LN patients for a given pathway-specific biologic treatment. Two mediators which are altered in the current assessment of SLE patients with renal involvement are SCF and TNFRII (Figs. 9A to 9E). SCF and its interaction with the receptor, c-kit, have previously been shown to contribute to kidney damage, including glomerulonephritis and renal failure (96, 97). TNFRII, normally present on lymphocytes (98-100), is aberrantly upregulated in the context of chronic inflammation, including in the kidney, contributing to inflammation, kidney damage and kidney failure (101-104).

The ability to detect changes in immune status as a reflection of clinical disease activity allows for improved disease surveillance and treatment, which could improve patient outcomes and reduce the pathogenic and socioeconomic burdens of SLE (29). A special technical effect of the present invention is the advantage of calculating a patient's LDAII that does not require cut-offs for each soluble mediator to establish positivity, and/or does not require a priori knowledge of the inflammatory pathways that contribute to disease activity in a particular patient. The present invention can be further validated in prospective, multiethnic studies in SLE clinical trials and disease management. Depending on the comprehensive clinical picture of an individual patient, early detection of risk for heightened clinical disease activity and organ damage prompts closer monitoring, the selection of one or more preventative treatments, or inclusion in clinical trials for targeted biologics relevant to pathways altered within the LDAII.

It will be understood that particular embodiments described herein are shown by way of illustration and not as limitations of the invention. The principal features of this invention can be employed in various embodiments without departing from the scope of the invention.

The use of the word "a" or "an" when used in conjunction with the term "comprising" in the claims and/or the specification may mean "one," but it is also consistent with the meaning of "one or more," "at least one," and "one or more than one." The use of the term "or" in the claims is used to mean "and/or" unless explicitly indicated to refer to alternatives only or the alternatives are mutually exclusive, although the disclosure supports a definition that refers to only alternatives and "and/or." Throughout this application, the term "about" is used to indicate that a value includes the inherent variation of error for the device, the method being employed to determine the value, or the variation that exists among the study subjects.

As used in this specification and claim(s), the words "comprising" (and any form of comprising, such as "comprise" and "comprises"), "having" (and any form of having, such as "have" and "has"), "including" (and any form of including, such as "includes" and "include") or "containing" (and any form of containing, such as "contains" and "contain") are inclusive or open-ended and do not exclude additional, unrecited elements or method steps. In embodiments of any of the compositions and methods provided herein, "comprising" may be replaced with "consisting essentially of' or "consisting of". As used herein, the phrase "consisting essentially of' requires the specified integer(s) or steps as well as those that do not materially affect the character or function of the claimed invention. As used herein, the term "consisting" is used to indicate the presence of the recited integer (e.g., a feature, an element, a characteristic, a property, a method/process step or a limitation) or group of integers (e.g., feature(s), element(s), characteristic(s), property(ies), method/process steps or limitation(s)) only.

The term "or combinations thereof" as used herein refers to all permutations and combinations of the listed items preceding the term. For example, "A, B, C, or combinations thereof' is intended to include at least one of: A, B, C, AB, AC, BC, or ABC, and if order is important in a particular context, also BA, CA, CB, CBA, BCA, ACB, BAC, or CAB. Continuing with this example, expressly included are combinations that contain repeats of one or more item or term, such as BB, AAA, AB, BBC, AAABCCCC, CBBAAA, CABABB, and so forth. The skilled artisan will understand that typically there is no limit on the number of items or terms in any combination, unless otherwise apparent from the context.

As used herein, words of approximation such as, without limitation, "about", "substantial" or "substantially" refers to a condition that when so modified is understood to not necessarily be absolute or perfect but would be considered close enough to those of ordinary skill in the art to warrant designating the condition as being present. The extent to which the description may vary will depend on how great a change can be instituted and still have one of ordinary skill in the art recognize the modified feature as still having the required characteristics and capabilities of the unmodified feature. In general, but subject to the preceding discussion, a numerical value herein that is modified by a word of approximation such as "about" may vary from the stated value by at least ±1, 2, 3, 4, 5, 6, 7, 10, 12 or 15%.

### REFERENCES

1. Lim SS, Bayakly AR, Helmick CG, Gordon C, Easley KA, Drenkard C. The incidence and prevalence of systemic lupus erythematosus, 2002-2004: The Georgia Lupus Registry. Arthritis & rheumatology. 2014;66(2):357-68.
2. Somers EC, Marder W, Cagnoli P, Lewis EE, DeGuire P, Gordon C, et al. Population-based incidence and prevalence of systemic lupus erythematosus: the Michigan Lupus Epidemiology and Surveillance program. Arthritis & rheumatology. 2014;66(2):369-78.
3. Tan EM, Cohen AS, Fries JF, Masi AT, McShane DJ, Rothfield NF, et al. The 1982 revised criteria for the classification of systemic lupus erythematosus. Arthritis and Rheumatism. 1982;25(11):1271-7.
4. Hochberg MC. Updating the American College of Rheumatology revised criteria for the classification of systemic lupus erythematosus. Arthritis and Rheumatism. 1997;40(9):1725.
5. Feldman CH, Hiraki LT, Liu J, Fischer MA, Solomon DH, Alarcon GS, et al. Epidemiology and sociodemographics of systemic lupus erythematosus and lupus nephritis among US adults with Medicaid coverage, 2000-2004. Arthritis Rheum. 2013;65(3):753-63.
6. Kasitanon N, Intaniwet T, Wangkaew S, Pantana S, Sukitawut W, Louthrenoo W. The clinically quiescent phase in early-diagnosed SLE patients: inception cohort study. Rheumatology (Oxford). 2015;54(5):868-75.
7. Oglesby A, Korves C, Laliberte F, Dennis G, Rao S, Suthoff ED, et al. Impact of early versus late systemic lupus erythematosus diagnosis on clinical and economic outcomes. Appl Health Econ Health Policy. 2014;12(2):179-90.
8. Thong B, Olsen NJ. Systemic lupus erythematosus diagnosis and management. Rheumatology (Oxford). 2017;56(suppl_1):i3-i13.
9. Conti F, Ceccarelli F, Perricone C, Leccese I, Massaro L, Pacucci VA, et al. The chronic damage in systemic lupus erythematosus is driven by flares, glucocorticoids and antiphospholipid antibodies: results from a monocentric cohort. Lupus. 2016;25(7):719-26.
10. Barral PM, Sarkar D, Su ZZ, Barber GN, DeSalle R, Racaniello VR, et al. Functions of the cytoplasmic RNA sensors RIG-I and MDA-5: key regulators of innate immunity. Pharmacol Ther. 2009;124(2):219-34.
11. Faurschou M, Starklint H, Halberg P, Jacobsen S. Prognostic factors in lupus nephritis: diagnostic and therapeutic delay increases the risk of terminal renal failure. J Rheumatol. 2006;33(8):1563-9.
12. Bruce IN, O'Keeffe AG, Farewell V, Hanly JG, Manzi S, Su L, et al. Factors associated with damage accrual in patients with systemic lupus erythematosus: results from the Systemic Lupus International Collaborating Clinics (SLICC) Inception Cohort. Ann Rheum Dis.2015;74(9):1706-13.
13. Alarcon GS, McGwin G, Jr., Petri M, Ramsey-Goldman R, Fessler BJ, Vila LM, et al. Time to renal disease and end-stage renal disease in PROFILE: a multiethnic lupus cohort. PLoS Med. 2006;3(10):e396.
14. Steiman AJ, Gladman DD, Ibanez D, Urowitz MB. Prolonged serologically active clinically quiescent systemic lupus erythematosus: frequency and outcome. J Rheumatol. 2010;37(9):1822-7.
15. Ng KP, Manson JJ, Rahman A, Isenberg DA. Association of antinucleosome antibodies with disease flare in serologically active clinically quiescent patients with systemic lupus erythematosus. Arthritis Rheum. 2006;55(6):900-4.
16. Petri M, Kim MY, Kalunian KC, Grossman J, Hahn BH, Sammaritano LR, et al. Combined oral contraceptives in women with systemic lupus erythematosus. N Engl J Med. 2005;353(24):2550-8.
17. Hay EM, Bacon PA, Gordon C, Isenberg DA, Maddison P, Snaith ML, et al. The BILAG index: a reliable and valid instrument for measuring clinical disease activity in systemic lupus erythematosus. Q J Med. 1993;86(7):447-58.
18. Lam GK, Petri M. Assessment of systemic lupus erythematosus. Clin Exp Rheumatol. 2005;23(5 Suppl 39):S120-32.
19. Peschken CA, Wang Y, Abrahamowicz M, Pope J, Silverman E, Sayani A, et al. Persistent Disease Activity Remains a Burden for Patients with Systemic Lupus Erythematosus. J Rheumatol. 2018.
20. Sheane BJ, Gladman DD, Su J, Urowitz MB. Disease Outcomes in Glucocorticosteroid-Naive Patients With Systemic Lupus Erythematosus. Arthritis care & research. 2017;69(2):252-6.
21. Zonana-Nacach A, Barr SG, Magder LS, Petri M. Damage in systemic lupus erythematosus and its association with corticosteroids. Arthritis Rheum. 2000;43(8):1801-8.
22. Thamer M, Hernan MA, Zhang Y, Cotter D, Petri M. Prednisone, lupus activity, and permanent organ damage. J Rheumatol. 2009;36(3):560-4.
23. Al Sawah S, Zhang X, Zhu B, Magder LS, Foster SA, Iikuni N, et al. Effect of corticosteroid use by dose on the risk of developing organ damage over time in systemic lupus erythematosus-the Hopkins Lupus Cohort. Lupus science & medicine. 2015;2(1):e000066.
24. Petri M, Purvey S, Fang H, Magder LS. Predictors of organ damage in systemic lupus erythematosus: the Hopkins Lupus Cohort. Arthritis Rheum. 2012;64(12):4021-8.
25. Lopez R, Davidson JE, Beeby MD, Egger PJ, Isenberg DA. Lupus disease activity and the risk of subsequent organ damage and mortality in a large lupus cohort. Rheumatology (Oxford). 2012;51(3):491-8.
26. Maroz N, Segal MS. Lupus nephritis and end-stage kidney disease. The American journal of the medical sciences. 2013;346(4):319-23.
27. Durcan L, Petri M. Why targeted therapies are necessary for systemic lupus erythematosus. Lupus. 2016;25(10):1070-9.
28. Doria A, Gatto M, Zen M, Iaccarino L, Punzi L. Optimizing outcome in SLE: treating-to-target and definition of treatment goals. Autoimmun Rev. 2014;13(7):770-7.
29. Lau CS, Mak A. The socioeconomic burden of SLE. Nat Rev Rheumatol. 2009;5(7):400-4.
30. Manzi S, Sanchez-Guerrero J, Merrill JT, Furie R, Gladman D, Navarra SV, et al. Effects of belimumab, a B lymphocyte stimulator-specific inhibitor, on disease activity across multiple organ domains in patients with systemic lupus erythematosus: combined results from two phase III trials. Ann Rheum Dis. 2012;71(11):1833-8.
31. Mikdashi J, Nived O. Measuring disease activity in adults with systemic lupus erythematosus: the challenges of administrative burden and responsiveness to patient concerns in clinical research. Arthritis Res Ther. 2015;17:183.
32. Thanou A, Chakravarty E, James JA, Merrill JT. Which outcome measures in SLE clinical trials best reflect medical judgment? Lupus science & medicine. 2014;1(1):e000005.
33. Thanou A, Stavrakis S, Dyer JW, Munroe ME, James JA, Merrill JT. Impact of heart rate variability, a marker for cardiac health, on lupus disease activity. Arthritis Res Ther. 2016;18:197.
34. Isenberg DA, Rahman A, Allen E, Farewell V, Akil M, Bruce IN, et al. BILAG 2004. Development and initial validation of an updated version of the British Isles Lupus Assessment Group's disease activity index for patients with systemic lupus erythematosus. Rheumatology (Oxford). 2005;44(7):902-6.
35. Mosca M, Tani C, Aringer M, Bombardieri S, Boumpas D, Cervera R, et al. Development of quality indicators to evaluate the monitoring of SLE patients in routine clinical practice. Autoimmun Rev. 2011;10(7):383-8.
36. Lu R, Munroe ME, Guthridge JM, Bean KM, Fife DA, Chen H, et al. Dysregulation of Innate and Adaptive Serum Mediators Precedes Systemic Lupus Erythematosus Classification and Improves Prognostic Accuracy of Autoantibodies J Autoimmun. 2016;74:182-93.
37. Munroe ME, Lu R, Zhao YD, Fife DA, Robertson JM, Guthridge JM, et al. Altered type II interferon precedes autoantibody accrual and elevated type I interferon activity prior to systemic lupus erythematosus classification. Ann Rheum Dis. 2016;75(11):2014-21.
38. Arriens C, Wren JD, Munroe ME, Mohan C. Systemic lupus erythematosus biomarkers: the challenging quest. Rheumatology (Oxford). 2016.
39. Crowe SR, Merrill JT, Vista ES, Dedeke AB, Thompson DM, Stewart S, et al. Influenza vaccination responses in human systemic lupus erythematosus: impact of clinical and demographic features. Arthritis Rheum. 2011;63(8):2396-406.
40. Kheir JM, Guthridge CJ, Johnston JR, Adams LJ, Rasmussen A, Gross TF, et al. Unique clinical characteristics, autoantibodies and medication use in Native American patients with systemic lupus erythematosus. Lupus science & medicine. 2018;5(1):e000247.
41. Munroe ME, Young KA, Kamen DL, Guthridge JM, Niewold TB, Costenbader KH, et al. Discerning Risk of Disease Transition in Relatives of Systemic Lupus Erythematosus Patients Utilizing Soluble Mediators and Clinical Features. Arthritis & rheumatology. 2017;69(3):630-42.
42. Munroe ME, Vista ES, Merrill JT, Guthridge JM, Roberts VC, James JA. Pathways of impending disease flare in African-American systemic lupus erythematosus patients. J Autoimmun. 2017;78:70-8.
43. Aberle T, Bourn RL, Munroe ME, Chen H, Roberts VC, Guthridge JM, et al. Clinical and serological features distinguish patients with incomplete lupus classification from systemic lupus erythematosus patients and controls. Arthritis care & research. 2017.
44. Slight-Webb S, Lu R, Ritterhouse LL, Munroe ME, Maecker HT, Fathman CG, et al. Autoantibody-Positive Healthy Individuals Display Unique Immune Profiles That May Regulate Autoimmunity. Arthritis & rheumatology. 2016;68(10):2492-502.
45. Munroe ME, Vista ES, Guthridge JM, Thompson LF, Merrill JT, James JA. Pro-inflammatory adaptive cytokines and shed tumor necrosis factor receptors are elevated preceding systemic lupus erythematosus disease flare. Arthritis & rheumatology. 2014;66(7):1888-99.
46. Stringer EA, Baker KS, Carroll IR, Montoya JG, Chu L, Maecker HT, et al. Daily cytokine fluctuations, driven by leptin, are associated with fatigue severity in chronic fatigue syndrome: evidence of inflammatory pathology. Journal of translational medicine. 2013;11(1):93.
47. Dupont NC, Wang K, Wadhwa PD, Culhane JF, Nelson EL. Validation and comparison of luminex multiplex cytokine analysis kits with ELISA: determinations of a panel of nine cytokines in clinical sample culture supernatants. J Reprod Immunol. 2005;66(2):175-91.
48. Dossus L, Becker S, Achaintre D, Kaaks R, Rinaldi S. Validity of multiplex-based assays for cytokine measurements in serum and plasma from "non-diseased" subjects: comparison with ELISA. J Immunol Methods. 2009;350(1-2):125-32.
49. Bruner BF, Guthridge JM, Lu R, Vidal G, Kelly JA, Robertson JM, et al. Comparison of autoantibody specificities between traditional and bead-based assays in a large, diverse collection of patients with systemic lupus erythematosus and family members. Arthritis Rheum.2012;64(11):3677-86.
50. Arbuckle MR, McClain MT, Rubertone MV, Scofield RH, Dennis GJ, James JA, et al. Development of autoantibodies before the clinical onset of systemic lupus erythematosus. N Engl J Med. 2003;349(16):1526-33.
51. Lopez P, Scheel-Toellner D, Rodriguez-Carrio J, Caminal-Montero L, Gordon C, Suarez A. Interferon-alpha-induced B-lymphocyte stimulator expression and mobilization in healthy and systemic lupus erthymatosus monocytes. Rheumatology (Oxford). 2014;53(12):2249-58.
52. Harigai M, Kawamoto M, Hara M, Kubota T, Kamatani N, Miyasaka N. Excessive production of IFN-gamma in patients with systemic lupus erythematosus and its contribution to induction of B lymphocyte stimulator/B cell-activating factor/TNF ligand superfamily-13B. J Immunol. 2008;181(3):2211-9.
53. Vincent FB, Morand EF, Schneider P, Mackay F. The BAFF/APRIL system in SLE pathogenesis. Nat Rev Rheumatol. 2014.
54. Kawamoto M, Harigai M, Hara M, Kawaguchi Y, Tezuka K, Tanaka M, et al. Expression and function of inducible co-stimulator in patients with systemic lupus erythematosus: possible involvement in excessive interferon-gamma and anti-double-stranded DNA antibody production. Arthritis Res Ther. 2006;8(3):R62.
55. Sim JH, Kim HR, Chang SH, Kim IJ, Lipsky PE, Lee J. Autoregulatory function of interleukin-10-producing pre-naive B cells is defective in systemic lupus erythematosus. Arthritis Res Ther. 2015;17:190.
56. Wang D, Huang S, Yuan X, Liang J, Xu R, Yao G, et al. The regulation of the Treg/Th17 balance by mesenchymal stem cells in human systemic lupus erythematosus. Cellular & molecular immunology. 2017;14(5):423-31.
57. Shah K, Lee WW, Lee SH, Kim SH, Kang SW, Craft J, et al. Dysregulated balance of Th17 and Th1 cells in systemic lupus erythematosus. Arthritis Res Ther. 2010;12(2):R53.
58. Borchers AT, Leibushor N, Naguwa SM, Cheema GS, Shoenfeld Y, Gershwin ME. Lupus nephritis: a critical review. Autoimmun Rev. 2012;12(2):174-94.
59. Mok CC, Ho LY, Cheung MY, Yu KL, To CH. Effect of disease activity and damage on quality of life in patients with systemic lupus erythematosus: a 2-year prospective study. Scand J Rheumatol. 2009;38(2):121-7.
60. Eder L, Urowitz MB, Gladman DD. Damage in lupus patients--what have we learned so far? Lupus. 2013;22(12):1225-31.
61. Polachek A, Gladman DD, Su J, Urowitz MB. Defining Low Disease Activity in Systemic Lupus Erythematosus. Arthritis care & research. 2017;69(7):997-1003.
62. Tokano Y, Morimoto S, Kaneko H, Amano H, Nozawa K, Takasaki Y, et al. Levels of IL-12 in the sera of patients with systemic lupus erythematosus (SLE)--relation to Th1- and Th2-derived cytokines. Clin Exp Immunol. 1999;116(1):169-73.
63. Gomez D, Correa PA, Gomez LM, Cadena J, Molina JF, Anaya JM. Th1/Th2 cytokines in patients with systemic lupus erythematosus: is tumor necrosis factor alpha protective? Semin Arthritis Rheum. 2004;33(6):404-13.
64. Mok MY, Wu HJ, Lo Y, Lau CS. The Relation of Interleukin 17 (IL-17) and IL-23 to Th1/Th2 Cytokines and Disease Activity in Systemic Lupus Erythematosus. J Rheumatol. 2010;37(10):2046-52.
65. Liu CC, Ahearn JM. The search for lupus biomarkers. Best practice & research Clinical rheumatology. 2009;23(4):507-23.
66. Petri MA, van Vollenhoven RF, Buyon J, Levy RA, Navarra SV, Cervera R, et al. Baseline Predictors of Systemic Lupus Erythematosus Flares: Data From the Combined Placebo Groups in the Phase III Belimumab Trials. Arthritis Rheum. 2013;65(8):2143-53.
67. Villegas-Zambrano N, Martinez-Taboada VM, Bolivar A, San Martin M, Alvarez L, Marin MJ, et al. Correlation between clinical activity and serological markers in a wide cohort of patients with systemic lupus erythematosus: an eight-year prospective study. Ann N Y Acad Sci. 2009;1173:60-6.
68. Chiche L, Jourde-Chiche N, Whalen E, Presnell S, Gersuk V, Dang K, et al. Modular transcriptional repertoire analyses of adults with systemic lupus erythematosus reveal distinct type I and type II interferon signatures. Arthritis & rheumatology. 2014;66(6):1583-95.
69. Rose T, Grutzkau A, Hirseland H, Huscher D, Dahnrich C, Dzionek A, et al. IFNalpha and its response proteins, IP-10 and SIGLEC-1, are biomarkers of disease activity in systemic lupus erythematosus. Ann Rheum Dis. 2013;72(10):1639-45.
70. Ko K, Koldobskaya Y, Rosenzweig E, Niewold TB. Activation of the Interferon Pathway is Dependent Upon Autoantibodies in African-American SLE Patients, but Not in European-American SLE Patients. Frontiers in immunology. 2013;4:309.
71. Kennedy WP, Maciuca R, Wolslegel K, Tew W, Abbas AR, Chaivorapol C, et al. Association of the interferon signature metric with serological disease manifestations but not global activity scores in multiple cohorts of patients with SLE. Lupus science & medicine. 2015;2(1):e000080.
72. Kim J, Gross JA, Dillon SR, Min JK, Elkon KB. Increased BCMA expression in lupus marks activated B cells, and BCMA receptor engagement enhances the response to TLR9 stimulation. Autoimmunity. 2011;44(2):69-81.
73. Schneider P. The role of APRIL and BAFF in lymphocyte activation. Curr Opin Immunol. 2005;17(3):282-9.
74. Roschke V, Sosnovtseva S, Ward CD, Hong JS, Smith R, Albert V, et al. BLyS and APRIL form biologically active heterotrimers that are expressed in patients with systemic immune-based rheumatic diseases. J Immunol. 2002;169(8):4314-21.
75. Gordon C, Wofsy D, Wax S, Li Y, Pena Rossi C, Isenberg D. Post-hoc analysis of the Phase II/III APRIL-SLE study: Association between response to atacicept and serum biomarkers including BLyS and APRIL. Arthritis & rheumatology. 2017;69(1):122-30.
76. Parodis I, Zickert A, Sundelin B, Axelsson M, Gerhardsson J, Svenungsson E, et al. Evaluation of B lymphocyte stimulator and a proliferation inducing ligand as candidate biomarkers in lupus nephritis based on clinical and histopathological outcome following induction therapy. Lupus science & medicine. 2015;2(1):e000061.
77. Morel J, Roubille C, Planelles L, Rocha C, Fernandez L, Lukas C, et al. Serum levels of tumour necrosis factor family members a proliferation-inducing ligand (APRIL) and B lymphocyte stimulator (BLyS) are inversely correlated in systemic lupus erythematosus. Ann Rheum Dis. 2009;68(6):997-1002.
78. Stohl W, Metyas S, Tan SM, Cheema GS, Oamar B, Roschke V, et al. Inverse association between circulating APRIL levels and serological and clinical disease activity in patients with systemic lupus erythematosus. Ann Rheum Dis. 2004;63(9):1096-103.
79. Salazar-Camarena DC, Ortiz-Lazareno PC, Cruz A, Oregon-Romero E, Machado-Contreras JR, Munoz-Valle JF, et al. Association of BAFF, APRIL serum levels, BAFF-R, TACI and BCMA expression on peripheral B-cell subsets with clinical manifestations in systemic lupus erythematosus. Lupus. 2016;25(6):582-92.
80. Tai YT, Lin L, Xing L, Cho SF, Yu T, Acharya C, et al. APRIL signaling via TACI mediates immunosuppression by T regulatory cells in multiple myeloma: therapeutic implications. Leukemia. 2018.
81. Davidson A. Targeting BAFF in autoimmunity. Curr Opin Immunol. 2010;22(6):732-9.
82. Day ES, Cachero TG, Qian F, Sun Y, Wen D, Pelletier M, et al. Selectivity of BAFF/BLyS and APRIL for binding to the TNF family receptors BAFFR/BR3 and BCMA. Biochemistry. 2005;44(6):1919-31.
83. Fujio K, Okamura T, Sumitomo S, Yamamoto K. Regulatory T cell-mediated control of autoantibody-induced inflammation. Frontiers in immunology. 2012;3:28.
84. Carter NA, Rosser EC, Mauri C. Interleukin-10 produced by B cells is crucial for the suppression of Th17/Th1 responses, induction of T regulatory type 1 cells and reduction of collagen-induced arthritis. Arthritis Res Ther. 2012;14(1):R32.
85. Okamoto A, Fujio K, Okamura T, Yamamoto K. Regulatory T-cell-associated cytokines in systemic lupus erythematosus. J Biomed Biotechnol. 2011;2011:463412.
86. Chen Q, Kim YC, Laurence A, Punkosdy GA, Shevach EM. IL-2 controls the stability of Foxp3 expression in TGF-beta-induced Foxp3+ T cells in vivo. J Immunol. 2011;186(11):6329-37.
87. Barreto M, Ferreira RC, Lourenco L, Moraes-Fontes MF, Santos E, Alves M, et al. Low frequency of CD4+CD25+ Treg in SLE patients: a heritable trait associated with CTLA4 and TGFbeta gene variants. BMC Immunol. 2009;10:5.
88. Atfy M, Amr GE, Elnaggar AM, Labib HA, Esh A, Elokely AM. Impact of CD4+CD25high regulatory T-cells and FoxP3 expression in the peripheral blood of patients with systemic lupus erythematosus. The Egyptian journal of immunology / Egyptian Association of Immunologists. 2009;16(1):117-26.
89. Llorente L, Zou W, Levy Y, Richaud-Patin Y, Wijdenes J, Alcocer-Varela J, et al. Role of interleukin 10 in the B lymphocyte hyperactivity and autoantibody production of human systemic lupus erythematosus. J Exp Med. 1995;181(3):839-44.
90. Striz I, Brabcova E, Kolesar L, Sekerkova A. Cytokine networking of innate immunity cells: a potential target of therapy. Clinical science. 2014;126(9):593-612.
91. Lee Y, Awasthi A, Yosef N, Quintana FJ, Xiao S, Peters A, et al. Induction and molecular signature of pathogenic TH17 cells. Nat Immunol. 2012;13(10):991-9.
92. Sarra M, Monteleone G. Interleukin-21: a new mediator of inflammation in systemic lupus erythematosus. J Biomed Biotechnol. 2010;2010:294582.
93. Nalbandian A, Crispin JC, Tsokos GC. Interleukin-17 and systemic lupus erythematosus: current concepts. Clin Exp Immunol. 2009;157(2):209-15.
94. Ibanez D, Gladman DD, Touma Z, Nikpour M, Urowitz MB. Optimal frequency of visits for patients with systemic lupus erythematosus to measure disease activity over time. J Rheumatol. 2011;38(1):60-3.
95. Ward MM, Sundaramurthy S, Lotstein D, Bush TM, Neuwelt CM, Street RL, Jr. Participatory patient-physician communication and morbidity in patients with systemic lupus erythematosus. Arthritis Rheum. 2003;49(6):810-8.
96. El-Koraie AF, Baddour NM, Adam AG, El Kashef EH, El Nahas AM. Role of stem cell factor and mast cells in the progression of chronic glomerulonephritides. Kidney Int.2001;60(1):167-72.
97. Kitoh T, Ishikawa H, Ishii T, Nakagawa S. Elevated SCF levels in the serum of patients with chronic renal failure. Br J Haematol. 1998;102(5):1151-6.
98. Kim EY, Priatel JJ, Teh SJ, Teh HS. TNF receptor type 2 (p75) functions as a costimulator for antigen-driven T cell responses in vivo. J Immunol. 2006;176(2):1026-35.
99. Kim EY, Teh HS. TNF type 2 receptor (p75) lowers the threshold of T cell activation. J Immunol. 2001;167(12):6812-20.
100. Munroe ME, Bishop GA. Role of tumor necrosis factor (TNF) receptor-associated factor 2 (TRAF2) in distinct and overlapping CD40 and TNF receptor 2/CD120b-mediated B lymphocyte activation. J Biol Chem. 2004;279(51):53222-31.
101. Speeckaert MM, Speeckaert R, Laute M, Vanholder R, Delanghe JR. Tumor necrosis factor receptors: biology and therapeutic potential in kidney diseases. American journal of nephrology. 2012;36(3):261-70.
102. Kurashina T, Nagasaka S, Watanabe N, Yabe D, Sugi N, Nin K, et al. Circulating TNF receptor 2 is closely associated with the kidney function in non-diabetic Japanese subjects. Journal of atherosclerosis and thrombosis. 2014;21(7):730-8.
103. Al-Lamki RS, Mayadas TN. TNF receptors: signaling pathways and contribution to renal dysfunction. Kidney Int. 2015;87(2):281-96.
104. Upadhyay A, Larson MG, Guo CY, Vasan RS, Lipinska I, O'Donnell CJ, et al. Inflammation, kidney function and albuminuria in the Framingham Offspring cohort. Nephrol Dial Transplant. 2011;26(3):920-6.

## Claims

1. A method for characterizing disease activity in a systemic lupus erythematosus patient (SLE), comprising:
(a) obtaining a dataset associated with a blood, serum, plasma or urine sample from the patient, wherein the dataset comprises data representing the level of one or more biomarkers in the blood, serum, plasma or urine sample from each of(b) to (g);
(b) assessing the dataset for a presence or an amount of protein expression of at least one innate serum or plasma soluble mediator biomarker selected from: IL-1α, IL-1β, IL-1RA, IFN-α, IL- 12p70, IL-6, and IL-23p19;
(c) assessing the dataset for a presence or an amount of protein expression of at least one adaptive serum or plasma soluble mediator biomarker selected from: IL-2, IFN-γ, IL-5, IL-13, IL-17A, IL-21, IL-10, and TGF-β;
(d) assessing the dataset for a presence or an amount of at least one chemokine/adhesion molecule soluble biomarker selected from: IL-8/CXCL8, IP-10/CXCL10, MIG/CXCL9, MIP-1α/CCL3, MIP-1β/CCL4, MCP-1/CCL2, MCP-3/CCL7, and ICAM-1;
(e) assessing the dataset for a presence or an amount of at least one soluble TNF superfamily biomarker selected from: TNFRI, TNFRII, TRAIL, TWEAK, CD40L/CD154, BLyS, and APRIL;
(f) assessing the dataset for a presence or an amount of the inflammatory soluble mediator biomarker SCF;
(g) assessing the dataset for a presence or an amount at least one SLE-associated autoantibody specificity biomarker selected from: dsDNA, chromatin, RiboP, Ro/SSA, La/SSB, Sm, SmRNP, and RNP; and
(h) calculating a Lupus Disease Activity Immune Index (LDAII) score;
wherein the dataset is: log transformed; standardized; weighted by Spearman r correlation to the autoantibody specificities, and a summation of the soluble protein markers equals the Lupus Disease Activity Immune Index (LDAII) score; wherein the LDAII score is calculated as follows:
the concentrations of the biomarkers are determined and log-transformed for the subject and each log-transformed soluble mediator level determined for the subject sample is standardized as follows:
(observed value)-(mean value of all SLE patients and healthy control visits)/(standard deviation of all SLE patient and healthy control visits);
generating Spearman coefficients from a linear regression model testing associations between one or more auto-antibody (AutoAb) specificities for each soluble mediator assessed in the SLE patient compared to healthy controls (Spearman r);
transforming and standardizing the values of the soluble mediator levels of the subject and the values weighted (multiplied) by their respective Spearman coefficients (Spearman r); and
summing for each participant visit, the log transformed, standardized and weighted values for each of the soluble mediators to calculate the LDAII.

2. A method of evaluating disease activity and progression of Systemic Lupus Erythematosus (SLE) clinical disease in a patient comprising:
performing at least one immunoassay on a sample that has been obtained from a patient, wherein the sample is a blood, serum, plasma or urine sample from the patient, to generate a dataset comprising at least one biomarker from each of (1) to (6):
(1) assessing the dataset for a presence or an amount of protein expression of at least one innate serum or plasma soluble mediator biomarker selected from: IL-1α, IL-1β, IL-1RA, IFN-α, IL- 12p70, IL-6, and IL-23p 19;
(2) assessing the dataset for a presence or an amount of protein expression of at least one adaptive serum or plasma soluble mediator biomarker selected from: IL-2, IFN-γ, IL-5, IL-13, IL-17A, IL-21, IL-10, and TGF-β;
(3) assessing the dataset for a presence or an amount of at least one chemokine/adhesion soluble molecule biomarker selected from: IL-8/CXCL8, IP-10/CXCL10, MIG/CXCL9, MIP-1α/CCL3, MIP-1β/CCL4, MCP-1/CCL2, MCP-3/CCL7, and ICAM-1;
(4) assessing the dataset for a presence or an amount of at least one soluble TNF superfamily biomarker selected from: TNFRI, TNFRII, TRAIL, TWEAK, CD40L/CD 154, BLyS, and APRIL;
(5) assessing the dataset for a presence or an amount of the inflammatory soluble mediator biomarker SCF; and
(6) assessing the dataset for a presence or an amount at least one SLE-associated autoantibody specificity biomarker selected from: dsDNA, chromatin, RiboP, Ro/SSA, La/SSB, Sm, SmRNP, and RNP;
and calculating an LDAII score;
wherein the dataset is: log transformed; standardized; weighted by Spearman r correlation to the autoantibody specificities, and a summation of the soluble protein markers equals the Lupus Disease Activity Immune Index (LDAII) score;
wherein the LDAII score is calculated as follows:
the concentration biomarkers are determined and log-transformed for the subject and each log-transformed soluble mediator level determined for the subject sample is standardized as follows:
(observed value)-(mean value of all SLE patients and healthy control visits)/(standard deviation of all SLE patient and healthy control visits);
generating Spearman coefficients from a linear regression model testing associations between one or more auto-antibody (AutoAb) specificities for each soluble mediator assessed in the SLE patient compared to healthy controls (Spearman r);
transforming and standardizing the values of the soluble mediator levels of the subject and the values weighted (multiplied) by their respective Spearman coefficients (Spearman r); and
summing for each participant visit, the log transformed, standardized and weighted values for each of the soluble mediators to calculate the LDAII.

3. The method of claim 2, wherein performance of the at least one immunoassay comprises: contacting a first sample with a plurality of distinct reagents, wherein the first sample has been obtained from the patient and comprises the protein markers; generating a plurality of distinct complexes between the reagents and markers; and detecting the complexes to generate the data.

4. The method of claim 1 or claim 2, wherein the LDAII divides a level of severity or progression of the SLE into clinically active (CA) or quiescent (CQ) disease that is either serologically (dsDNA binding and low complement) active (SA) or serologically quiescent (SQ).

5. The method of claim 1 or claim 2, wherein the LDAII score distinguishes between active and low lupus disease activity.

6. The method of claim 2, wherein obtaining the first dataset associated with the sample comprises processing the sample obtained from the patient to experimentally determine the first dataset, or wherein obtaining the first dataset associated with the sample comprises receiving the first dataset from a third party that has processed the sample to experimentally determine the first dataset.

7. The method of claim 2, wherein an increase in the SCF, TNFRII, and MCP-1 biomarkers are indicative of renal organ involvement.

8. A method of calculating a Lupus Disease Activity Immune Index (LDAII) by measuring expression levels of a set of biomarkers in a subject comprising:
determining biomarker measures of a set of biomarkers by immunoassay in a physiological sample, wherein the biomarkers are peptides, proteins, peptides bearing post-translational modifications, proteins bearing post-translational modification, or a combination thereof; wherein the physiological sample is whole blood, blood plasma, blood serum, or a combination thereof; wherein the set of biomarkers comprise a dataset of measurements selected from at least one from each category of biomarkers (b) to (g):
(b) a presence or an amount of protein expression of at least one innate serum or plasma soluble mediator biomarker dataset selected from: IL-1α, IL-1β, IL-1RA, IFN-α, IL-12p70, IL-6, and IL-23p 19;
(c) a presence or an amount of protein expression of at least one adaptive serum or plasma soluble mediator biomarker dataset selected from: IL-2, IFN-γ, IL-5, IL-13, IL-17A, IL-21, IL-10, and TGF-β;
(d) a presence or an amount of at least one chemokine/adhesion molecule soluble biomarker dataset selected from: IL-8/CXCL8, IP-10/CXCL10, MIG/CXCL9, MIP-1α/CCL3, MIP-1β/CCL4, MCP-1/CCL2, MCP-3/CCL7, and ICAM-1;
(e) a presence or an amount of at least one soluble TNF superfamily biomarker dataset selected from: TNFRI, TNFRII, TRAIL, TWEAK, CD40L/CD154, BLyS, and APRIL;
(f) a presence or an amount of the inflammatory soluble mediator biomarker SCF; and
(g) a presence or an amount at least one SLE-associated autoantibody specificity biomarker dataset selected from: dsDNA, chromatin, RiboP, Ro/SSA, La/SSB, Sm, SmRNP, and RNP;
and calculating an LDAII score, whereby the dataset is: log transformed; standardized; weighted by Spearman r correlation to the autoantibody specificities, and a summation of the soluble protein markers equals an LDAII score;
wherein the LDAII score is calculated as follows:
the concentration biomarkers are determined and log-transformed for the subject and each log-transformed soluble mediator level determined for the subject sample is standardized as follows:
(observed value)-(mean value of all SLE patients and healthy control visits)/(standard deviation of all SLE patient and healthy control visits);
generating Spearman coefficients from a linear regression model testing associations between one or more auto-antibody (AutoAb) specificities for each soluble mediator assessed in the SLE patient compared to healthy controls (Spearman r);
transforming and standardizing the values of the soluble mediator levels of the subject and the values weighted (multiplied) by their respective Spearman coefficients (Spearman r); and
summing for each participant visit, the log transformed, standardized and weighted values for each of the soluble mediators to calculate the LDAII.

9. The method of claim 8, further comprising classifying the sample with respect to the presence or development of Systemic Lupus Erythematosus (SLE) into clinically active (CA) or quiescent (CQ) disease that is either serologically (dsDNA binding and low complement) active (SA) or serologically quiescent (SQ) in the subject using the set of biomarker measures in a classification system, wherein the classification system is a machine learning system comprising classification and regression trees selected from the group consisting of Fisher's exact test, Mann-Whitney test, Kruskal-Wallis test, Kruskal-Wallis test with Dunn's multiple comparison, Spearman's rank correlation or an ensemble thereof; and
calculating the Lupus Disease Activity Immune Index (LDAII), wherein the LDAII score distinguishes between active SLE and low SLE disease activity (low clinical disease (SLEDAI < 4); or
further comprising differentiating clinically and serologically quiescent (CQSQ) SLE patients compared to healthy controls.

10. The method of any of claims 1, 2 or 8, further comprising determining an amount of at least 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, or 39 biomarkers cited in said claims in the calculation of the LDAII.

11. The method of claims 2 or 8, wherein the immunoassay is a multiplexed immunoassay.

12. The method of claim 8, wherein an increase in the LDAII is indicative of at least one of SLE disease progression, increased autoimmune disease activity, or organ damage.

## Patentansprüche

1. Verfahren zum Charakterisieren einer Krankheitsaktivität bei einem Patienten mit systemischem Lupus erythematodes (SLE), umfassend:
a) Erhalten eines Datensatzes, der mit einer Blut-, Serum-, Plasma- oder Urinprobe des Patienten assoziiert ist, wobei der Datensatz Daten umfasst, die den Spiegel eines oder mehrerer Biomarker in der Blut-, Serum-, Plasma- oder Urinprobe aus jedem der Punkte (b) bis (g) darstellen;
b) Untersuchen des Datensatzes auf ein Vorhandensein oder eine Menge einer Proteinexpression mindestens eines löslichen angeborenen Serum- oder Plasma-Mediator-Biomarkers, ausgewählt aus: IL-1α, IL-1β, IL-1RA, IFN-α, IL-12p70, IL-6 und IL-23p19;
c) Untersuchen des Datensatzes auf ein Vorhandensein oder eine Menge einer Proteinexpression mindestens eines löslichen adaptiven Serum- oder Plasma-Mediator-Biomarkers, ausgewählt aus: IL-2, IFN-γ, IL-5, IL-13, IL-17A, IL-21, IL-10 und TGF-β;
d) Untersuchen des Datensatzes auf ein Vorhandensein oder eine Menge mindestens eines löslichen Chemokin/Adhäsionsmolekül-Biomarkers, ausgewählt aus: IL-8/CXCL8, IP-10/CXCL10, MIG/CXCL9, MIP-1α/CCL3, MIP-1β/CCL4, MCP-1/CCL2, MCP-3/CCL7 und ICAM-1;
e) Untersuchen des Datensatzes auf ein Vorhandensein oder eine Menge mindestens eines löslichen Biomarkers der TNF-Superfamilie, ausgewählt aus: TNFRI, TNFRII, TRAIL, TWEAK, CD40L/CD154, BLyS und APRIL;
f) Untersuchen des Datensatzes auf ein Vorhandensein oder eine Menge des löslichen Entzündungsmediator-Biomarkers SCF;
g) Untersuchen des Datensatzes auf ein Vorhandensein oder eine Menge mindestens eines SLE-assoziierten Autoantikörper-Spezifitätsbiomarkers, ausgewählt aus: dsDNA, Chromatin, RiboP, Ro/SSA, La/SSB, Sm, SmRNP und RNP; und
h) Berechnen eines Lupus Disease Activity Immune Index- (LDAII)-Scores;
wobei der Datensatz: logarithmisch transformiert; standardisiert; mit der Spearman-r-Korrelation zu den Autoantikörperspezifitäten gewichtet ist und eine Summe der löslichen Proteinmarker dem Lupus Disease Activity Immune Index- (LDAII)-Score entspricht; wobei der LDAII-Score wie folgt berechnet wird:
die Konzentrationen der Biomarker werden für das Subjekt bestimmt und logarithmisch transformiert, und jeder für die Subjektprobe bestimmte, logarithmisch transformierte Spiegel an löslichem Mediator wird wie folgt standardisiert:
(beobachteter Wert)-(Mittelwert aller Visiten bei SLE-Patienten und gesunden Kontrollpersonen)/(Standardabweichung aller Visiten bei SLE-Patienten und gesunden Kontrollpersonen);
Erzeugen von Spearman-Koeffizienten aus einem linearen Regressionsmodell, das Assoziationen zwischen einer oder mehreren Autoantikörperspezifitäten (AutoAb-Spezifitäten) für jeden löslichen Mediator, der beim SLE-Patienten untersucht wurde, im Vergleich zu gesunden Kontrollpersonen testet (Spearman r);
Transformieren und Standardisieren der Werte der löslichen Mediatorspiegel des Subjekts und der mit ihren jeweiligen Spearman-Koeffizienten (Spearman r) gewichteten (multiplizierten) Werte;
und für jede Teilnehmervisite Summieren der logarithmisch transformierten, standardisierten und gewichteten Werte für jeden der löslichen Mediatoren, um den LDAII zu berechnen.

2. Verfahren zum Bewerten einer Krankheitsaktivität und eines Fortschreitens der klinischen Erkrankung systemischer Lupus erythematodes (SLE) bei einem Patienten, umfassend:
Durchführen mindestens eines Immunassays an einer Probe, die von einem Patienten erhalten wurde, wobei die Probe eine Blut-, Serum-, Plasma- oder Urinprobe des Patienten ist, um einen Datensatz zu erzeugen, der mindestens einen Biomarker aus jedem der Punkte (1) bis (6) umfasst:
(1) Untersuchen des Datensatzes auf ein Vorhandensein oder eine Menge einer Proteinexpression mindestens eines löslichen angeborenen Serum- oder Plasma-Mediator-Biomarkers, ausgewählt aus: IL-1α, IL-1β, IL-1RA, IFN-α, IL-12p70, IL-6 und IL-23p19;
(2) Untersuchen des Datensatzes auf ein Vorhandensein oder eine Menge einer Proteinexpression mindestens eines löslichen adaptiven Serum- oder Plasma-Mediator-Biomarkers, ausgewählt aus: IL-2, IFN-γ, IL-5, IL-13, IL-17A, IL-21, IL-10 und TGF-β;
(3) Untersuchen des Datensatzes auf ein Vorhandensein oder eine Menge mindestens eines löslichen Chemokin/Adhäsionsmolekül-Biomarkers ausgewählt aus: IL-8/CXCL8, IP-10/CXCL10, MIG/CXCL9, MIP-1α/CCL3, MIP-1β/CCL4, MCP-1/CCL2, MCP-3/CCL7 und ICAM-1;
(4) Untersuchen des Datensatzes auf ein Vorhandensein oder eine Menge mindestens eines löslichen Biomarkers der TNF-Superfamilie, ausgewählt aus: TNFRI, TNFRII, TRAIL, TWEAK, CD40L/CD154, BLyS und APRIL;
(5) Untersuchen des Datensatzes auf ein Vorhandensein oder eine Menge des löslichen Entzündungsmediator-Biomarkers SCF; und
(6) Untersuchen des Datensatzes auf ein Vorhandensein oder eine Menge mindestens eines SLE-assoziierten Autoantikörper-Spezifitätsbiomarkers, ausgewählt aus: dsDNA, Chromatin, RiboP, Ro/SSA, La/SSB, Sm, SmRNP und RNP;
und Berechnen eines LDAII-Scores;
wobei der Datensatz: logarithmisch transformiert; standardisiert; mit der Spearman-r-Korrelation zu den Autoantikörperspezifitäten gewichtet ist und eine Summe der löslichen Proteinmarker dem Lupus Disease Activity Immune Index- (LDAII)-Score entspricht;
wobei der LDAII-Score wie folgt berechnet wird:
die Biomarkerkonzentrationen werden für das Subjekt bestimmt und logarithmisch transformiert, und jeder für die Subjektprobe bestimmte, logarithmisch transformierte Spiegel an löslichem Mediator wird wie folgt standardisiert:
(beobachteter Wert)-(Mittelwert aller Visiten bei SLE-Patienten und gesunden Kontrollpersonen)/(Standardabweichung aller Visiten bei SLE-Patienten und gesunden Kontrollpersonen);
Erzeugen von Spearman-Koeffizienten aus einem linearen Regressionsmodell, das Assoziationen zwischen einer oder mehreren Autoantikörperspezifitäten (AutoAb-Spezifitäten) für jeden löslichen Mediator, der beim SLE-Patienten untersucht wurde, im Vergleich zu gesunden Kontrollpersonen testet (Spearman r);
Transformieren und Standardisieren der Werte der löslichen Mediatorspiegel des Subjekts und der mit ihren jeweiligen Spearman-Koeffizienten (Spearman r) gewichteten (multiplizierten) Werte; und
Summieren für jede Teilnehmervisite der logarithmisch transformierten, standardisierten und gewichteten Werte für jeden der löslichen Mediatoren, um den LDAII zu berechnen.

3. Verfahren nach Anspruch 2, wobei Durchführung des mindestens einen Immunassays umfasst: Kontaktieren einer ersten Probe mit einer Vielzahl unterschiedlicher Reagenzien, wobei die erste Probe von dem Patienten erhalten wurde und die Proteinmarker umfasst;
Erzeugen einer Vielzahl unterschiedlicher Komplexe zwischen den Reagenzien und Markern und Erfassen der Komplexe, um die Daten zu erzeugen.

4. Verfahren nach Anspruch 1 oder Anspruch 2, wobei der LDAII einen Schweregrad oder ein Fortschreiten des SLE in klinisch aktive (CA) oder ruhende (CQ) Krankheit unterteilt, die entweder serologisch (dsDNA-Bindung und geringes Komplement) aktiv (SA) oder serologisch ruhend (SQ) ist.

5. Verfahren nach Anspruch 1 oder Anspruch 2, wobei der LDAII-Score zwischen aktiver und geringer Lupus-Krankheitsaktivität unterscheidet.

6. Verfahren nach Anspruch 2, wobei Erhalten des mit der Probe assoziierten ersten Datensatzes Verarbeiten der von dem Patienten erhaltenen Probe umfasst, um den ersten Datensatz experimentell zu bestimmen, oder wobei Erhalten des mit der Probe assoziierten ersten Datensatzes Empfangen des ersten Datensatzes von einem Dritten umfasst, der die Probe verarbeitet hat, um den ersten Datensatz experimentell zu bestimmen.

7. Verfahren nach Anspruch 2, wobei ein Anstieg der Biomarker SCF, TNFRII und MCP-1 auf eine Nierenbeteiligung hinweist.

8. Verfahren zum Berechnen eines Lupus Disease Activity Immune Index (LDAII) durch Messen von Expressionsspiegeln eines Satzes von Biomarkern in einem Subjekt, umfassend:
Bestimmen von Biomarkermengen aus einem Satz von Biomarkern durch Immunassay in einer physiologischen Probe, wobei die Biomarker Peptide, Proteine, Peptide mit posttranslationalen Modifikationen, Proteine mit posttranslationaler Modifikation oder eine Kombination davon sind; wobei die physiologische Probe Vollblut, Blutplasma, Blutserum oder eine Kombination davon ist; wobei der Satz von Biomarkern einen Datensatz von Messungen umfasst, ausgewählt aus mindestens einer aus jeder Kategorie von Biomarkern (b) bis (g):
b) ein Vorhandensein oder eine Menge einer Proteinexpression mindestens eines Datensatzes eines löslichen angeborenen Serum- oder Plasma-Mediator-Biomarkers, ausgewählt aus: IL-1α, IL-1β, IL-1RA, IFN-α, IL-12p70, IL-6 und IL-23p19;
c) ein Vorhandensein oder eine Menge einer Proteinexpression mindestens eines Datensatzes eines löslichen adaptiven Serum- oder Plasma-Mediator-Biomarkers, ausgewählt aus: IL-2, IFN-γ, IL-5, IL-13, IL-17A, IL-21, IL-10 und TGF-β;
d) ein Vorhandensein oder eine Menge mindestens eines Datensatzes eines löslichen Chemokin/Adhäsionsmolekül-Biomarkers, ausgewählt aus: IL-8/CXCL8, IP-10/CXCL10, MIG/CXCL9, MIP-1α/CCL3, MIP-1β/CCL4, MCP-1/CCL2, MCP-3/CCL7, und ICAM-1;
e) ein Vorhandensein oder eine Menge mindestens eines Datensatzes eines löslichen Biomarkers derTNF-Superfamilie, ausgewählt aus: TNFRI, TNFRII, TRAIL, TWEAK, CD40L/CD154, BLyS und APRIL;
f) ein Vorhandensein oder eine Menge des löslichen Entzündungsmediator-Biomarkers SCF; und
g) ein Vorhandensein oder eine Menge mindestens eines Datensatzes eines SLE-assoziierten Autoantikörper-Spezifitätsbiomarkers, ausgewählt aus: dsDNA, Chromatin, RiboP, Ro/SSA, La/SSB, Sm, SmRNP und RNP;
und Berechnen eines LDAII-Scores, wobei der Datensatz: logarithmisch transformiert; standardisiert; mit der Spearman-r-Korrelation zu den Autoantikörperspezifitäten gewichtet ist und eine Summe der löslichen Proteinmarker einem LDAII-Score entspricht;
wobei der LDAII-Score wie folgt berechnet wird:
die Biomarkerkonzentrationen werden für das Subjekt bestimmt und logarithmisch transformiert, und jeder für die Subjektprobe bestimmte, logarithmisch transformierte Spiegel an löslichem Mediator wird wie folgt standardisiert:
(beobachteter Wert)-(Mittelwert aller Visiten bei SLE-Patienten und gesunden Kontrollpersonen)/(Standardabweichung aller Visiten bei SLE-Patienten und gesunden Kontrollpersonen);
Erzeugen von Spearman-Koeffizienten aus einem linearen Regressionsmodell, das Assoziationen zwischen einer oder mehreren Autoantikörperspezifitäten (AutoAb-Spezifitäten) für jeden löslichen Mediator, der beim SLE-Patienten untersucht wurde, im Vergleich zu gesunden Kontrollpersonen testet (Spearman r);
Transformieren und Standardisieren der Werte der löslichen Mediatorspiegel des Subjekts und der mit ihren jeweiligen Spearman-Koeffizienten (Spearman r) gewichteten (multiplizierten) Werte; und
Summieren für jede Teilnehmervisite der logarithmisch transformierten, standardisierten und gewichteten Werte für jeden der löslichen Mediatoren, um den LDAII zu berechnen.

9. Verfahren nach Anspruch 8, das weiter Klassifizieren der Probe in Bezug auf das Vorhandensein oder die Entwicklung von systemischem Lupus erythematodes (SLE) in klinisch aktive (CA) oder ruhende (CQ) Krankheit, die entweder serologisch (dsDNA-Bindung und geringes Komplement) aktiv (SA) oder serologisch ruhend (SQ) ist, beim Subjekt umfasst, unter Verwendung des Satzes von Biomarkermengen in einem Klassifikationssystem, wobei das Klassifikationssystem ein maschinelles Lernsystem ist, das Klassifikations- und Regressionsbäume umfasst, die aus der Gruppe ausgewählt sind, die aus Fishers exaktem Test, Mann-Whitney-Test, Kruskal-Wallis-Test, Kruskal-Wallis-Test mit Dunns Mehrfachvergleich, Spearmans Rangkorrelation oder einem Ensemble davon besteht; und
Berechnen des Lupus Disease Activity Immune Index (LDAII), wobei der LDAII-Score zwischen aktivem SLE und geringer SLE-Krankheitsaktivität (niedrige klinische Krankheitsaktivität (SLEDAI < 4)) unterscheidet; oder
weiter umfassend Unterscheiden zwischen klinisch und serologisch ruhenden (CQSQ) SLE-Patienten im Vergleich zu gesunden Kontrollpersonen.

10. Verfahren nach einem der Ansprüche 1, 2 oder 8, das weiter Bestimmen einer Menge von mindestens 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38 oder 39 der in den genannten Ansprüchen genannten Biomarker bei der Berechnung des LDAII umfasst.

11. Verfahren nach den Ansprüchen 2 oder 8, wobei der Immunassay ein Multiplex-Immunassay ist.

12. Verfahren nach Anspruch 8, wobei ein Anstieg des LDAII auf mindestens eines der folgenden Anzeichen hinweist: Fortschreiten der SLE-Erkrankung, erhöhte Aktivität der Autoimmunerkrankung oder Organschaden.

## Revendications

1. Procédé pour caractériser une activité de maladie chez un patient atteint de lupus érythémateux disséminé (LED), comprenant les étapes consistant à :
a) obtenir un ensemble de données associé à un échantillon de sang, de sérum, de plasma ou d'urine du patient, dans lequel l'ensemble de données comprend des données représentant le niveau d'un ou de plusieurs biomarqueurs dans l'échantillon de sang, de sérum, de plasma ou d'urine de chacun des points (b) à (g) ;
b) évaluer l'ensemble de données pour déterminer une présence ou une quantité d'expression protéique d'au moins un biomarqueur de médiateur naturel soluble dans le sérum ou le plasma sélectionné parmi : IL-1α, IL-1β, IL-1RA, IFN-α, IL-12p70, IL-6 et IL-23p19 ;
c) évaluer l'ensemble de données pour déterminer une présence ou une quantité d'expression protéique d'au moins un biomarqueur de médiateur adaptatif soluble dans le sérum ou le plasma sélectionné parmi : IL-2, IFN-γ, IL-5, IL-13, IL-17A, IL-21, IL-10 et TGF-β ;
d) évaluer l'ensemble de données pour une présence ou une quantité d'au moins un biomarqueur soluble de chimiokine/molécule d'adhérence sélectionné parmi : IL-8/CXCL8, IP-10/CXCL10, MIG/CXCL9, MIP-1α/CCL3, MIP-1β/CCL4, MCP-1/CCL2, MCP-3/CCL7, et ICAM-1 ;
e) évaluer l'ensemble de données pour déterminer une présence ou une quantité d'au moins un biomarqueur soluble de la superfamille des TNF sélectionné parmi : TNFRI, TNFRII, TRAIL, TWEAK, CD40L/CD154, BLyS et AVRIL ;
f) évaluer l'ensemble de données pour une présence ou une quantité du biomarqueur soluble de médiateur inflammatoire SCF ;
g) évaluer l'ensemble de données pour déterminer une présence ou une quantité d'au moins un biomarqueur de spécificité d'auto-anticorps associé au LED sélectionné parmi : ADNds, chromatine, RiboP, Ro/SSA, La/SSB, Sm, SmRNP et RNP ; et
h) calculer un score d'indice immunitaire d'activité de maladie de lupus (LDAII) ;
dans lequel l'ensemble de données est : soumis à une transformation logarithmique ; standardisé ; pondéré par une corrélation de Spearman r avec les spécificités d'auto-anticorps, et une somme de marqueurs protéiques solubles est égale au score d'indice immunitaire d'activité de maladie de lupus (LDAII); dans lequel le score LDAII est calculé comme suit :
les concentrations des biomarqueurs sont déterminées et soumises à une transformation logarithmique pour le sujet et chaque niveau de médiateur soluble soumis à une transformation logarithmique déterminé pour l'échantillon sujet est standardisé comme suit :
(valeur observée)-(valeur moyenne de tous les patients atteints de LED et visiteurs témoins sains)/(écart type de tous les patients atteints de LED et visiteurs témoins sains );
générer des coefficients de Spearman à partir d'un modèle de régression linéaire testant des associations entre une ou plusieurs spécificités d'auto-anticorps (AutoAb) pour chaque médiateur soluble évalué chez le patient atteint de LED par rapport à des témoins sains (Spearman r) ;
transformer et standardiser les valeurs des niveaux de médiateur soluble du sujet et les valeurs pondérées (multipliées) par leurs coefficients de Spearman respectifs (Spearman r) ;
et additionner pour chaque visiteur participant, les valeurs soumises à une transformation logarithmique, standardisées et pondérées pour chacun des médiateurs solubles afin de calculer le LDAII.

2. Procédé d'évaluation d'une activité de maladie et d'une progression d'une maladie clinique du lupus érythémateux disséminé (LED) chez un patient, comprenant les étapes consistant à :
effectuer au moins un test immunologique sur un échantillon qui a été obtenu auprès d'un patient, dans lequel l'échantillon est un échantillon de sang, de sérum, de plasma ou d'urine provenant du patient pour générer un ensemble de données comprenant au moins un biomarqueur de chacune des étapes (1) à ( 6) consistant à :
(1) évaluer l'ensemble de données pour déterminer une présence ou une quantité d'expression protéique d'au moins un biomarqueur de médiateur naturel soluble dans le sérum ou le plasma sélectionné parmi : IL-1α, IL-1β, IL-1RA, IFN-α, IL-12p70, IL-6 et IL-23p19 ;
(2) évaluer l'ensemble de données pour déterminer une présence ou une quantité d'expression protéique d'au moins un biomarqueur de médiateur adaptatif soluble dans le sérum ou le plasma sélectionné parmi : IL-2, IFN-γ, IL-5, IL-13, IL-17A, IL-21, IL-10 et TGF-β ;
(3) évaluer l'ensemble de données pour une présence ou une quantité d'au moins un biomarqueur soluble de chimiokine/molécule d'adhérence sélectionné parmi : IL-8/CXCL8, IP-10/CXCL10, MIG/CXCL9, MIP-1α/CCL3, MIP-1β/CCL4, MCP-1/CCL2, MCP-3/CCL7, et ICAM-1 ;
(4) évaluer l'ensemble de données pour déterminer une présence ou une quantité d'au moins un biomarqueur soluble de la superfamille des TNF sélectionné parmi : TNFRI, TNFRII, TRAIL, TWEAK, CD40L/CD154, BLyS et APRIL;
(5) évaluer l'ensemble de données pour une présence ou une quantité du biomarqueur soluble de médiateur inflammatoire SCF ; et
(6) évaluer l'ensemble de données pour déterminer une présence ou une quantité d'au moins un biomarqueur de spécificité d'auto-anticorps associé au SEL sélectionné parmi : ADNds, chromatine, RiboP, Ro/SSA, La/SSB, Sm, SmRNP et RNP ;
et calculer un score LDAII ;
dans lequel l'ensemble de données est : soumis à une transformation logarithmique ; standardisé ; pondéré par une corrélation de Spearman r avec les spécificités d'auto-anticorps, et une somme de marqueurs protéiques solubles est égale au score d'indice immunitaire d'activité de maladie de lupus (LDAII) ;
dans lequel le score LDAII est calculé comme suit :
les concentrations des biomarqueurs sont déterminées et soumises à une transformation logarithmique pour le sujet et chaque niveau de médiateur soluble soumis à une transformation logarithmique déterminé pour l'échantillon sujet est standardisé comme suit :
(valeur observée)-(valeur moyenne de tous les patients atteints de LED et visiteurs témoins sains)/(écart type de tous les patients atteints de LED et visiteurs témoins sains );
générer des coefficients de Spearman à partir d'un modèle de régression linéaire testant des associations entre une ou plusieurs spécificités d'auto-anticorps (AutoAb) pour chaque médiateur soluble évalué chez le patient atteint de LED par rapport à des témoins sains (Spearman r) ;
transformer et standardiser les valeurs des niveaux de médiateur soluble du sujet et les valeurs pondérées (multipliées) par leurs coefficients de Spearman respectifs (Spearman r) ; et
additionner pour chaque visiteur participant, les valeurs soumises à une transformation logarithmique, standardisées et pondérées pour chacun des médiateurs solubles afin de calculer le LDAII.

3. Procédé selon la revendication 2, dans lequel la réalisation du au moins un test immunologique comprend les étapes consistant à : mettre en contact un premier échantillon avec une pluralité de réactifs distincts, dans lequel le premier échantillon a été obtenu auprès du patient et comprenant les marqueurs protéiques ;
générer une pluralité de complexes distincts entre les réactifs et les marqueurs ; et détecter les complexes pour générer les données.

4. Procédé selon la revendication 1 ou la revendication 2, dans lequel le LDAII divise un niveau de gravité ou de progression du LED en une maladie cliniquement active (CA) ou quiescente (CQ) qui est soit sérologiquement (liaison d'ADNds et faible complément) active (SA) soit sérologiquement quiescente (SQ).

5. Procédé selon la revendication 1 ou la revendication 2, dans lequel le score LDAII fait la distinction entre une activité de maladie de lupus active et faible.

6. Procédé selon la revendication 2, dans lequel l'obtention du premier ensemble de données associé à l'échantillon comprend un traitement de l'échantillon obtenu auprès du patient pour déterminer expérimentalement le premier ensemble de données, ou dans lequel l'obtention du premier ensemble de données associé à l'échantillon comprend une réception du premier ensemble de données d'un tiers qui a traité l'échantillon pour déterminer expérimentalement le premier ensemble de données.

7. Procédé selon la revendication 2, dans lequel une augmentation des biomarqueurs SCF, TNFRII et MCP-1 indique une atteinte des organes rénaux.

8. Procédé de calcul d'un indice immunitaire d'activité de maladie de lupus (LDAII) en mesurant des niveaux d'expression d'un ensemble de biomarqueurs chez un sujet, comprenant les étapes consistant à :
déterminer des mesures de biomarqueur d'un ensemble de biomarqueurs par dosage immunologique dans un échantillon physiologique, dans lequel les biomarqueurs sont des peptides, des protéines, des peptides portant des modifications post-traductionnelles, des protéines portant des modifications post-traductionnelles, ou une combinaison de ceux-ci ; dans lequel l'échantillon physiologique est du sang total, du plasma sanguin, du sérum sanguin ou une combinaison de ceux-ci ; dans lequel l'ensemble de biomarqueurs comprend un ensemble de données de mesures sélectionnées parmi au moins une de chaque catégorie de biomarqueurs (b) à (g) :
b) une présence ou une quantité d'expression protéique d'au moins un ensemble de données biomarqueur de médiateur naturel soluble dans le sérum ou le plasma sélectionné parmi : IL-1α, IL-1β, IL-1RA, IFN-α, IL-12p70, IL-6 et IL-23p19 ;
c) une présence ou une quantité d'expression protéique d'au moins un ensemble de données de biomarqueur de médiateur adaptatif solubles dans le sérum ou le plasma sélectionné parmi : IL-2, IFN-γ, IL-5, IL-13, IL-17A, IL-21, IL-10 et TGF-β ;
d) une présence ou une quantité d'au moins un ensemble de données de biomarqueur soluble de chimiokine/molécule d'adhérence sélectionné parmi : IL-8/CXCL8, IP-10/CXCL10, MIG/CXCL9, MIP-1α/CCL3, MIP-1β/CCL4, MCP-1/CCL2, MCP-3/CCL7, et ICAM-1 ;
e) évaluer l'ensemble de données pour déterminer une présence ou une quantité d'au moins un ensemble de données de biomarqueur soluble de la superfamille des TNF sélectionné parmi : TNFR!, TNFRII, TRAIL, TWEAK, CD40L/CD154, BLyS et APRIL;
f) une présence ou une quantité du biomarqueur soluble de médiateur inflammatoire SCF ; et
(6) une présence ou une quantité d'au moins un ensemble de données de biomarqueur de spécificité d'auto-anticorps associé au LED sélectionné parmi : ADNds, chromatine, RiboP, Ro/SSA, La/SSB, Sm, SmRNP et RNP ;
et calculer un score LDAII, l'ensemble de données étant : soumis à une transformation logarithmique ; standardisé ; pondéré par une corrélation de Spearman r avec les spécificités d'auto-anticorps, et une somme des marqueurs protéiques solubles équivaut à un score LDAII ;
dans lequel le score LDAII est calculé comme suit :
les concentrations des biomarqueurs sont déterminées et soumises à une transformation logarithmique pour le sujet et chaque niveau de médiateur soluble soumis à une transformation logarithmique déterminé pour l'échantillon sujet est standardisé comme suit :
(valeur observée)-(valeur moyenne de tous les patients atteints de LED et visiteurs témoins sains)/(écart type de tous les patients atteints de LED et visiteurs témoins sains );
générer des coefficients de Spearman à partir d'un modèle de régression linéaire testant des associations entre une ou plusieurs spécificités d'auto-anticorps (AutoAb) pour chaque médiateur soluble évalué chez le patient atteint de LED par rapport à des témoins sains (Spearman r) ;
transformer et standardiser les valeurs des niveaux de médiateur soluble du sujet et les valeurs pondérées (multipliées) par leurs coefficients de Spearman respectifs (Spearman r) ; et
additionner pour chaque visiteur participant, les valeurs soumises à une transformation logarithmique, standardisées et pondérées pour chacun des médiateurs solubles afin de calculer le LDAII

9. Procédé selon la revendication 8, comprenant en outre une classification de l'échantillon en ce qui concerne la présence ou le développement du lupus érythémateux systémique (LED) en une maladie cliniquement active (CA) ou quiescente (CQ) qui est soit sérologiquement (liaison à de l'ADNds et faible complément) active (SA) ou sérologiquement quiescente (SQ) chez le sujet à l'aide de l'ensemble de mesures de biomarqueur dans un système de classification, dans lequel le système de classification est un système d'apprentissage automatique comprenant des arbres de classification et de régression sélectionnés parmi le groupe consistant en le test exact de Fisher et le test de Mann-Whitney, du test de Kruskal-Wallis, le test de Kruskal-Wallis avec comparaison multiple de Dunn, une corrélation de rang de Spearman ou un ensemble de ceux-ci ; et
calculer l'indice immunitaire d'activité de maladie de lupus (LDAII), dans lequel le score LDAII fait la distinction entre le LED actif et une faible activité de maladie LED (SLEDA! de maladie clinique faible < 4) ; ou
comprenant en outre une différenciation de patients atteints de LED cliniquement et sérologiquement quiescent (CQSQ) par comparaison avec des témoins sains.

10. Procédé selon l'une quelconque des revendications 1, 2 ou 8, comprenant en outre une détermination d'une quantité d'au moins 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38 ou 39 biomarqueurs cités dans lesdites revendications dans le calcul du LDAII.

11. Procédé selon l'une quelconque des revendications 2 à 8, dans lequel le dosage immunologique est un dosage immunologique multiplexé.

12. Procédé selon la revendication 8, dans lequel une augmentation du LDAII est indicative d'au moins une parmi : une progression de la maladie LED, une activité accrue de maladie auto-immune ou une lésion d'un organe.
